# EUROPEAN PATENT APPLICATION

(11) **EP 4 130 265 A1**
(43) Date of publication of application: **08.02.2023**
(21) Application number: 21780839.3
(22) Date of filing: 01.04.2021
(51) Int. Cl.: C12N 15/11, C12Q 1/689

(54) **METHOD FOR DETERMINING SINGLE BASE MUTATION OF ERM (41) GENE OF ACID-FAST BACILLUS BELONGING TO MYCOBACTEROIDES ABSCESSUS COMPLEX, AND PRIMER SET AND PROBE USED IN SAID METHOD**

(30) Priority: 01.04.2020 JP 2020066277
(71) Applicant: Japan as Represented by Director-General of National Institute of Infectious Diseases, Tokyo 162-8640 (JP); Kaneka Corporation, Osaka-shi, Osaka 530-8288 (JP)
(72) Inventor: HOSHINO ,Yoshihiko, Higashimurayama-shi, Tokyo 189-0002 (JP); YOSHIDA ,Mitsunori, Higashimurayama-shi, Tokyo 189-0002 (JP); SANO, Sotaro, Takasago-shi, Hyogo 676-8688 (JP); MIYAMOTO, Shigehiko, Takasago-shi, Hyogo 676-8688 (JP)
(74) Representative: Hoffmann Eitle
(86) International application number: PCT/JP2021/014128
(87) International publication number: WO 2021/201206

(57) **Abstract**

According to one or more embodiments of the present invention, there is provided a new means for determining a single nucleotide mutation in erm (41) gene in a mycobacterium belonging to *Mycobacteroides abscessus* complex.

One or more embodiments of the present invention relate to a method for determining the base corresponding to position 28 in erm (41) gene in a mycobacterium belonging to *Mycobacteroides abscessus* complex, comprising
detecting, in a genomic DNA of the mycobacterium as a subject to be determined, an indicator nucleotide sequence, which is present in the region except erm (41) gene in the genomic DNA when the base in erm (41) gene is cytosine, and which is not present when the base is thymine, wherein the presence of the indicator nucleotide sequence indicates that the base is cytosine, and the absence of the indicator nucleotide sequence indicates that the base is thymine.

## Description

### Technical Field

The present invention relates to a method for determining a single nucleotide mutation in erm (41) gene of a mycobacterium (acid-fast bacillus) belonging to *Mycobacteroides abscessus* complex, and a primer set and probe for use in the method. The present invention also relates to a method for determining a single nucleotide mutation in erm (41) gene of mycobacterium, and a primer set and probe for use in the method.

### Background Art

Mycobacterium is an anti-acidic, non-motile gram-positive bacillus. Mycobacterium is generally classified into two groups: Mycobacterium tuberculosis complex; and Nontuberculous mycobacteria (NTM).

Up to present, 170 species or more of NTM have been reported. About 30 species of them have been known to be pathogenic to humans.

Of infections with NTM, infection with *Mycobacteroides abscessus* complex is hard-to-treat with existing antibacterial drugs, and an effective treatment has not yet been established. Because of this, the number of patients has grown and the number of serious cases has increased. A causative bacterial group, *Mycobacteroides abscessus* complex, has three subspecies: *Mycobacteroides abscessus* subsp. *abscessus, Mycobacteroides abscessus* subsp. *massiliense* and *Mycobacteroides abscessus* subsp. *bolletii.* Note that, *Mycobacteroides abscessus* is sometimes referred to as *Mycobacterium abscessus.*

Patent Literature 1 discloses a primer set for distinguishing these three subspecies of *Mycobacteroides abscessus* complex. The primer set contains a first primer, which targets a region downstream of a membrane transporter gene commonly present in three subspecies and is designed so as to provide amplified products different in size among the subspecies; and a second primer, which targets a region downstream of an ATP binding cassette transporter gene commonly present in the three subspecies and is designed so as to provide amplified products different in size among the subspecies.

In *Mycobacteroides abscessus* complex, it is known that resistance to an antibiotic substance is developed by mutation of a gene. In *Mycobacteroides abscessus* complex, there are a strain sensitive to macrolide and a strain inducing tolerance on medication thereof. A single nucleotide polymorphism in erm (41) gene is involved in the presence or absence of induced tolerance. It is known that a T28 strain in which the base at position 28 in erm (41) gene is thymine has induced tolerance, whereas, a C28 strain in which the base at position 28 is cytosine has a sensitivity to a drug (see, Non Patent Literature 1).

A method for detecting a mutant of a bacterium, such as *Mycoplasma pneumoniae*, having resistance to a macrolide antibiotic is disclosed in Patent Literature 2. In the method, a hydrolytic probe, which shows different behavior between a standard strain and a mutant strain, is brought into contact with a biological sample of a test subject and detection is performed by real-time PCR.

For determining a single nucleotide polymorphism of a gene, techniques using a primer extension reaction (including nucleic acid amplification) by an allele-specific primer is known. The allele-specific primer refers to a primer exhibiting a significantly different extension efficiency depending on a type of a nucleotide at a target single nucleotide polymorphism. Accordingly, if PCR is performed using the allele-specific primer and the amount of an amplified product is analyzed, the type of a nucleotide at a target single nucleotide polymorphism can be specified. However, in this case, unless a reaction condition is strictly specified, a false-positive problem may occur. Patent Literature 3 discloses a method for determining a single nucleotide polymorphism having few false-positive reactions using an allele specific primer having a specific pattern at the second and third nucleotides from the 3'-end and the 3'-end nucleotide corresponding to a target single nucleotide polymorphism. However, in any case of designing an allele-specific primer for directly determining a single nucleotide polymorphism, it is necessary to provide a site corresponding to a single nucleotide polymorphism site near the 3' end of the primer. Because of this, flexibility for designing such a primer is low. In addition, depending on a surrounding sequence of a single nucleotide polymorphism, it is difficult to select a primer having an extension efficiency sufficient for determination and few false-positive reactions.

### Citation List

### Patent Literatures

Patent Literature 1: JP Patent Publication (Kokai) No. 2019-97493A
Patent Literature 2: WO2013/136818
Patent Literature 3: JP Patent No. 3859678

### Non Patent Literature

Non Patent Literature 1: J Antimicrob Chemother 2017; 72: 1669-1677

### Summary of Invention

### Technical Problem

As described above, a single nucleotide polymorphism in erm (41) gene is involved in the presence or absence of macrolide induced tolerance of *Mycobacteroides abscessus* complex. More specifically, it is known that a T28 strain in which the base at position 28 in erm (41) gene is thymine has induced tolerance, whereas, a C28 strain in which the base at position 28 is cytosine has a sensitivity. Then, for estimating macrolide sensitivity of *Mycobacteroides abscessus* complex, it is useful to distinguish whether *Mycobacteroides abscessus* complex is a T28 strain or a C28 strain.

To obtain an allele-specific primer for directly detecting a single nucleotide polymorphism, it is necessary to design a primer having a sufficient extension efficiency for determination and few false-positive reactions as disclosed in Patent Literature 3 from limited candidate sequences. It is not easy to design such a primer.

According to one or more embodiments of the present invention, there is provided a new means for determining whether the base at position 28 in erm (41) gene is cytosine or thymine in a mycobacterium belonging to *Mycobacteroides abscessus* complex. According to one or more embodiments of the present invention, there is provided a new means for determining whether the base at position 28 in erm (41) gene is cytosine in a mycobacterium.

### Solution to Problem

The present inventors surprisingly found an indicator nucleotide sequence, which is present when the base at position 28 in erm (41) gene on a genomic DNA of a mycobacterium belonging to *Mycobacteroides abscessus* complex is cytosine and which is not present when the base at position 28 is thymine. They found that the base at position 28 in erm (41) gene of a mycobacterium as a subject to be determined can be determined based on the presence or absence of the indicator nucleotide sequence. Based on the findings, the following inventions were accomplished.
[1] A method for determining whether the base corresponding to position 28 in the nucleotide sequence of SEQ ID NO:1 of erm (41) gene is cytosine or thymine in a mycobacterium belonging to *Mycobacteroides abscessus* complex, comprising
   detecting, in a genomic DNA of the mycobacterium as a subject to be determined, an indicator nucleotide sequence, which is present in the region except the erm (41) gene in the genomic DNA when the base in the erm (41) gene is cytosine, and which is not present when the base is thymine, wherein
   detection of the indicator nucleotide sequence indicates that the base in the erm (41) gene in the genomic DNA of the mycobacterium is cytosine, and
   non-detection of the indicator nucleotide sequence indicates that the base in the erm (41) gene in the genomic DNA of the mycobacterium is thymine.
[2] The method according to [1], wherein the indicator nucleotide sequence is a first nucleotide sequence having consecutive 10 or more nucleotides contained in the nucleotide sequence of SEQ ID NO:2, or a second nucleotide sequence complementary to the first nucleotide sequence.
[3] The method according to [2], wherein the first nucleotide sequence at least partially comprises a nucleotide sequence having consecutive 10 or more nucleotides contained in a nucleotide sequence of SEQ ID NO:3, 5, 7, 9 or 11.
[4] A method for determining sensitivity of a mycobacterium belonging to *Mycobacteroides abscessus* complex to a macrolide antibiotic substance, comprising the method according to any one of [1] to [3], wherein
   detection of the indicator nucleotide sequence indicates that the mycobacterium has a sensitivity to a macrolide antibiotic substance, and
   non-detection of the indicator nucleotide sequence indicates that the mycobacterium does not have a sensitivity to a macrolide antibiotic substance.
[5] A primer set for detecting an indicator nucleotide sequence, which is present in the region except erm (41) gene of a genomic DNA when the base corresponding to position 28 in the nucleotide sequence of SEQ ID NO:1 of erm (41) gene is cytosine and which is not present when the base is thymine, in a mycobacterium belonging to *Mycobacteroides abscessus* complex, the primer set comprising:
   a first primer comprising a polynucleotide comprising, at the 3' end, a nucleotide sequence f12 capable of hybridizing to a complementary nucleotide sequence to a partial nucleotide sequence f11 having consecutive 10 or more nucleotides which is contained in the indicator nucleotide sequence, and
   a second primer comprising a polynucleotide comprising, at the 3' end, a nucleotide sequence r12 capable of hybridizing to a partial nucleotide sequence r11 having consecutive 10 or more nucleotides which is contained in the indicator nucleotide sequence, and positioned closer to the 3' end than the 3' end of the partial nucleotide sequence f11.
[6] The primer set according to [5], wherein the indicator nucleotide sequence is a third nucleotide sequence having consecutive 20 or more nucleotides contained in the nucleotide sequence of SEQ ID NO:2.
[7] The primer set according to [6], wherein a nucleotide sequence from the nucleotide at the 5' end of the partial nucleotide sequence f11 to the nucleotide at the 3' end of the partial nucleotide sequence r11 contained in the third nucleotide sequence at least partially comprises a nucleotide sequence having consecutive 20 or more nucleotides contained in a nucleotide sequence of SEQ ID NO:3, 5, 7, 9 or 11.
[8] The primer set according to [6] or [7], wherein
   the partial nucleotide sequence f11 is
      (f11-12-1) a nucleotide sequence having consecutive 10 or more nucleotides and contained in the nucleotide sequence in the range from position 4624 to position 4668 of the nucleotide sequence of SEQ ID NO:2,
      (f11-18-1) a nucleotide sequence having consecutive 10 or more nucleotides and contained in the nucleotide sequence in the range from position 2356 to position 2397 of the nucleotide sequence of SEQ ID NO:2,
      (f11-20-1) a nucleotide sequence having consecutive 10 or more nucleotides and contained in the nucleotide sequence in the range from position 2624 to position 2663 of the nucleotide sequence of SEQ ID NO:2,
      (f11-22-1) a nucleotide sequence having consecutive 10 or more nucleotides and contained in the nucleotide sequence in the range from position 4044 to position 4083 of the nucleotide sequence of SEQ ID NO:2,
      (f11-24-1) a nucleotide sequence having consecutive 10 or more nucleotides and contained in the nucleotide sequence in the range from position 6535 to position 6575 of the nucleotide sequence of SEQ ID NO:2,
      (f11-26-1) a nucleotide sequence having consecutive 10 or more nucleotides and contained in the nucleotide sequence in the range from position 7478 to position 7520 of the nucleotide sequence of SEQ ID NO:2, or
      (f11-28-1) a nucleotide sequence having consecutive 10 or more nucleotides and contained in the nucleotide sequence in the range from position 7629 to position 7673 of the nucleotide sequence of SEQ ID NO:2, and
   the partial nucleotide sequence r11 is
      (r11-13-1) a nucleotide sequence having consecutive 10 or more nucleotides and contained in the nucleotide sequence in the range from position 4694 to position 4740 of the nucleotide sequence of SEQ ID NO:2,
      (r 11-19-1) a nucleotide sequence having consecutive 10 or more nucleotides and contained in the nucleotide sequence in the range from position 2672 to position 2711 of the nucleotide sequence of SEQ ID NO:2,
      (r11-21-1) a nucleotide sequence having consecutive 10 or more nucleotides and contained in the nucleotide sequence in the range from position 3539 to position 3583 of the nucleotide sequence of SEQ ID NO:2,
      (r11-23-1) a nucleotide sequence having consecutive 10 or more nucleotides and contained in the nucleotide sequence in the range from position 5061 to position 5103 of the nucleotide sequence of SEQ ID NO:2,
      (r11-25-1) a nucleotide sequence having consecutive 10 or more nucleotides and contained in the nucleotide sequence in the range from position 6566 to position 6605 of the nucleotide sequence of SEQ ID NO:2,
      (r11-27-1) a nucleotide sequence having consecutive 10 or more nucleotides and contained in the nucleotide sequence in the range from position 7931 to position 7976 of the nucleotide sequence of SEQ ID NO:2, or
      (r11-29-1) a nucleotide sequence having consecutive 10 or more nucleotides and contained in the nucleotide sequence in the range from position 8302 to position 8346 of the nucleotide sequence of SEQ ID NO:2.
[9] The primer set according to any one of [5] to [8], wherein one of the first primer and the second primer further comprises a label portion that is a tag capable of binding to a label substance or a label substance, and the other one further comprises a binding portion that is a tag capable of binding to a solid phase support.
[10] A kit for detecting an indicator nucleotide sequence, which is present in the region except erm (41) gene of a genomic DNA when the base corresponding to position 28 in the nucleotide sequence of SEQ ID NO:1 of erm (41) gene is cytosine, and which is not present when the base is thymine, in a mycobacterium belonging to *Mycobacteroides abscessus* complex, the kit comprising:
   the primer set according to [9]; and
   a solid phase support at least partially comprising a portion capable of binding to the binding portion.
[11] A probe for detecting an indicator nucleotide sequence, which is present in the region except erm (41) gene of a genomic DNA when the base corresponding to position 28 in the nucleotide sequence of SEQ ID NO: 1 of erm (41) gene is cytosine and which is not present when the base is thymine, in a mycobacterium belonging to *Mycobacteroides abscessus* complex,
   the probe comprising a polynucleotide comprising a nucleotide sequence capable of hybridizing to a partial nucleotide sequence p1 having consecutive 10 or more nucleotides and contained in the indicator nucleotide sequence.
[12] The probe according to [11], wherein the indicator nucleotide sequence is a first nucleotide sequence having consecutive 10 or more nucleotides contained in the nucleotide sequence of SEQ ID NO:2, or a second nucleotide sequence complementary to the first nucleotide sequence.
[13] The probe according to [12], wherein the partial nucleotide sequence p1 at least partially comprises a nucleotide sequence having consecutive 10 or more nucleotides contained in a nucleotide sequence of SEQ ID NO:3, 5, 7, 9 or 11 or a complementary nucleotide sequence thereof.
[14] The probe according to [12] or [13], wherein the partial nucleotide sequence p1 is
   (p1-12-1) a nucleotide sequence having consecutive 10 or more nucleotides and contained in the nucleotide sequence in the range from position 4624 to position 4668 of the nucleotide sequence of SEQ ID NO:2 or a complementary nucleotide sequence thereof,
   (p1-18-1) a nucleotide sequence having consecutive 10 or more nucleotides and contained in the nucleotide sequence in the range from position 2356 to position 2397 of the nucleotide sequence of SEQ ID NO:2 or a complementary nucleotide sequence thereof,
   (p1-20-1) a nucleotide sequence having consecutive 10 or more nucleotides and contained in the nucleotide sequence in the range from position 2624 to position 2663 of the nucleotide sequence of SEQ ID NO:2 or a complementary nucleotide sequence thereof,
   (p1-22-1) a nucleotide sequence having consecutive 10 or more nucleotides and contained in the nucleotide sequence in the range from position 4044 to position 4083 of the nucleotide sequence of SEQ ID NO:2 or a complementary nucleotide sequence thereof,
   (p1-24-1) a nucleotide sequence having consecutive 10 or more nucleotides and contained in the nucleotide sequence in the range from position 6535 to position 6575 of the nucleotide sequence of SEQ ID NO:2 or a complementary nucleotide sequence thereof,
   (p1-26-1) a nucleotide sequence having consecutive 10 or more nucleotides and contained in the nucleotide sequence in the range from position 7478 to position 7520 of the nucleotide sequence of SEQ ID NO:2 or a complementary nucleotide sequence thereof,
   (p1-28-1) a nucleotide sequence having consecutive 10 or more nucleotides and contained in the nucleotide sequence in the range from position 7629 to position 7673 of the nucleotide sequence of SEQ ID NO:2 or a complementary nucleotide sequence thereof,
   (p1-13-1) a nucleotide sequence having consecutive 10 or more nucleotides and contained in the nucleotide sequence in the range from position 4694 to position 4740 of the nucleotide sequence of SEQ ID NO:2 or a complementary nucleotide sequence thereof,
   (p1-19-1) a nucleotide sequence having consecutive 10 or more nucleotides and contained in the nucleotide sequence in the range from position 2672 to position 2711 of the nucleotide sequence of SEQ ID NO:2 or a complementary nucleotide sequence thereof,
   (p1-21-1) a nucleotide sequence having consecutive 10 or more nucleotides and contained in the nucleotide sequence in the range from position 3539 to position 3583 of the nucleotide sequence of SEQ ID NO:2 or a complementary nucleotide sequence thereof,
   (p1-23-1) a nucleotide sequence having consecutive 10 or more nucleotides and contained in the nucleotide sequence in the range from position 5061 to position 5103 of the nucleotide sequence of SEQ ID NO:2 or a complementary nucleotide sequence thereof,
   (p1-25-1) a nucleotide sequence having consecutive 10 or more nucleotides and contained in the nucleotide sequence in the range from position 6566 to position 6605 of the nucleotide sequence of SEQ ID NO:2 or a complementary nucleotide sequence thereof,
   (p1-27-1) a nucleotide sequence having consecutive 10 or more nucleotides and contained in the nucleotide sequence in the range from position 7931 to position 7976 of the nucleotide sequence of SEQ ID NO:2 or a complementary nucleotide sequence thereof, or
   (p1-29-1) a nucleotide sequence having consecutive 10 or more nucleotides and contained in the nucleotide sequence in the range from position 8302 to position 8346 of the nucleotide sequence of SEQ ID NO:2 or a complementary nucleotide sequence thereof.
[15] A method for determining whether the base corresponding to position 28 in the nucleotide sequence of SEQ ID NO:1 of erm (41) gene is cytosine or thymine in a mycobacterium belonging to *Mycobacteroides abscessus* complex, comprising
   performing a nucleic acid amplification reaction using a genomic DNA of the mycobacterium as a subject to be determined or a polynucleotide which is derived from the genomic DNA of the mycobacterium as a subject to be determined, as a template, and using the primer set according to any one of [5] to [9],
   detecting an amplified product obtained by the nucleic acid amplification reaction, and
   determining that the base in erm (41) gene in the genomic DNA of the mycobacterium is cytosine when the amplified product is detected, and that the base is thymine when the amplified product is not detected.
[16] A method for determining sensitivity of a mycobacterium belonging to *Mycobacteroides abscessus* complex to a macrolide antibiotic substance, comprising the method according to [15], comprising
   determining that the mycobacterium has a sensitivity to a macrolide antibiotic substance when the amplified product is detected, and that the mycobacterium does not have a sensitivity to a macrolide antibiotic substance when the amplified product is not detected.
[17] A method for determining whether the base corresponding to position 28 in the nucleotide sequence of SEQ ID NO:1 of erm (41) gene is cytosine or thymine, in a mycobacterium belonging to *Mycobacteroides abscessus* complex, comprising
   incubating a genomic DNA of the mycobacterium as a subject to be determined or a polynucleotide derived from the genomic DNA of the mycobacterium as a subject to be determined, with the probe according to any one of [11] to [14], in a condition allowing hybridization,
   detecting hybridization of the genomic DNA or the polynucleotide and the probe, and
   determining that the base in erm (41) gene of the genomic DNA of the mycobacterium as the subject to be determined is cytosine when the hybridization is detected, and that the base is thymine when the hybridization is not detected.
[18] A method for determining sensitivity of a mycobacterium belonging to *Mycobacteroides abscessus* complex to a macrolide antibiotic substance, comprising the method according to [17], comprising
   determining that the mycobacterium has a sensitivity to a macrolide antibiotic substance when the hybridization is detected, and that the mycobacterium does not have a sensitivity to a macrolide antibiotic substance when the hybridization is not detected.
[19] A method for determining that the base corresponding to position 28 in the nucleotide sequence of SEQ ID NO:1 of erm (41) gene is cytosine in a mycobacterium, comprising
   detecting, in a genomic DNA of the mycobacterium as a subject to be determined, an indicator nucleotide sequence, which is present in the region except erm (41) gene in the genomic DNA when the base in erm (41) gene is cytosine, and which is not present when the base is thymine, wherein
   detection of the indicator nucleotide sequence indicates that the base in erm (41) gene in the genomic DNA of the mycobacterium as the subject to be determined is cytosine.
[20] The method according to [19], wherein the indicator nucleotide sequence is a first nucleotide sequence having consecutive 10 or more nucleotides contained in the nucleotide sequence of SEQ ID NO:2, or a second nucleotide sequence complementary to the first nucleotide sequence.
[21] The method according to [20], wherein the first nucleotide sequence at least partially comprises a nucleotide sequence having consecutive 10 or more nucleotides contained in a nucleotide sequence of SEQ ID NO:3, 5, 7, 9 or 11.
[22] A method for determining sensitivity of a mycobacterium to a macrolide antibiotic substance, comprising the method according to any one of [19] to [21], wherein
   detection of the indicator nucleotide sequence indicates that the mycobacterium has a sensitivity to a macrolide antibiotic substance.
[23] A primer set for detecting an indicator nucleotide sequence, which is present in the region except erm (41) gene of a genomic DNA when the base corresponding to position 28 in the nucleotide sequence of SEQ ID NO:1 of erm (41) gene is cytosine, and which is not present when the base is thymine, in a mycobacterium, the primer set comprising:
   a first primer comprising a polynucleotide comprising, at the 3' end, a nucleotide sequence f12 capable of hybridizing to a complementary nucleotide sequence to a partial nucleotide sequence f11 having consecutive 10 or more nucleotides which is contained in the indicator nucleotide sequence, and
   a second primer comprising a polynucleotide comprising, at the 3' end, a nucleotide sequence r12 capable of hybridizing to a partial nucleotide sequence r11 having consecutive 10 or more nucleotides which is contained in the indicator nucleotide sequence and positioned closer to the 3' end than the 3' end of the partial nucleotide sequence f11 and
[24] The primer set according to [23], wherein the indicator nucleotide sequence is a third nucleotide sequence having consecutive 20 or more nucleotides and contained in the nucleotide sequence of SEQ ID NO:2.
[25] The primer set according to [24], wherein a nucleotide sequence from the nucleotide at the 5' end of the partial nucleotide sequence f11 to the nucleotide at the 3' end of the partial nucleotide sequence r11 and contained in the third nucleotide sequence at least partially comprises a nucleotide sequence having consecutive 20 or more nucleotides contained in the nucleotide sequence of SEQ ID NO:3, 5, 7, 9 or 11.
[26] The primer set according to [24] or [25], wherein
   the partial nucleotide sequence f11 is:
      (f11-12-1) a nucleotide sequence having consecutive 10 or more nucleotides and contained in the nucleotide sequence in the range from position 4624 to position 4668 of the nucleotide sequence of SEQ ID NO:2,
      (f11-18-1) a nucleotide sequence having consecutive 10 or more nucleotides and contained in the nucleotide sequence in the range from position 2356 to position 2397 of the nucleotide sequence of SEQ ID NO:2,
      (f11-20-1) a nucleotide sequence having consecutive 10 or more nucleotides and contained in the nucleotide sequence in the range from position 2624 to position 2663 of the nucleotide sequence of SEQ ID NO:2,
      (f11-22-1) a nucleotide sequence having consecutive 10 or more nucleotides and contained in the nucleotide sequence in the range from position 4044 to position 4083 of the nucleotide sequence of SEQ ID NO:2,
      (f11-24-1) a nucleotide sequence having consecutive 10 or more nucleotides and contained in the nucleotide sequence in the range from position 6535 to position 6575 of the nucleotide sequence of SEQ ID NO:2,
      (f11-26-1) a nucleotide sequence having consecutive 10 or more nucleotides and contained in the nucleotide sequence in the range from position 7478 to position 7520 of the nucleotide sequence of SEQ ID NO:2, or
      (f11-28-1) a nucleotide sequence having consecutive 10 or more nucleotides and contained in the nucleotide sequence in the range from position 7629 to position 7673 of the nucleotide sequence of SEQ ID NO:2, and
   the partial nucleotide sequence r11 is
      (r11-13-1) a nucleotide sequence having consecutive 10 or more nucleotides and contained in the nucleotide sequence in the range from position 4694 to position 4740 of the nucleotide sequence of SEQ ID NO:2,
      (r 11-19-1) a nucleotide sequence having consecutive 10 or more nucleotides and contained in the nucleotide sequence in the range from position 2672 to position 2711 of the nucleotide sequence of SEQ ID NO:2,
      (r11-21-1) a nucleotide sequence having consecutive 10 or more nucleotides and contained in the nucleotide sequence in the range from position 3539 to position 3583 of the nucleotide sequence of SEQ ID NO:2,
      (r11-23-1) a nucleotide sequence having consecutive 10 or more nucleotides, in the nucleotide sequence in the range from position 5061 to position 5103 of the nucleotide sequence of SEQ ID NO:2,
      (r11-25-1) a nucleotide sequence having consecutive 10 or more nucleotides and contained in the nucleotide sequence in the range from position 6566 to position 6605 of the nucleotide sequence of SEQ ID NO:2,
      (r11-27-1) a nucleotide sequence having consecutive 10 or more nucleotides and contained in the nucleotide sequence in the range from position 7931 to position 7976 of the nucleotide sequence of SEQ ID NO:2, or
      (r11-29-1) a nucleotide sequence having consecutive 10 or more nucleotides and contained in the nucleotide sequence in the range from position 8302 to position 8346 of the nucleotide sequence of SEQ ID NO:2.
[27] The primer set according to any one of [23] to [26], wherein one of the first primer and the second primer further comprises a label portion that is a tag capable of binding to a label substance or a label substance, and the other one further comprises a binding portion that is a tag capable of binding to a solid phase support.
[28] A kit for detecting an indicator nucleotide sequence, which is present in the region except erm (41) gene of a genomic DNA when the base corresponding to position 28 in the nucleotide sequence of SEQ ID NO:1 of erm (41) gene is cytosine, and which is not present when the base is thymine, in a mycobacterium, the kit comprising:
   the primer set according to [27]; and
   a solid phase support at least partially comprising a portion capable of binding to the binding portion.
[29] A probe for detecting an indicator nucleotide sequence, which is present in the region except erm (41) gene of a genomic DNA when the base corresponding to position 28 in the nucleotide sequence of SEQ ID NO: 1 of erm (41) gene is cytosine and which is not present when the nucleotide is thymine, in a mycobacterium,
   the probe comprising a polynucleotide comprising a nucleotide sequence capable of hybridizing to a partial nucleotide sequence p1 having consecutive 10 or more nucleotides contained in the indicator nucleotide sequence.
[30] The probe according to [29], wherein the indicator nucleotide sequence is a first nucleotide sequence having consecutive 10 or more nucleotides contained in the nucleotide sequence of SEQ ID NO:2, or a second nucleotide sequence complementary to the first nucleotide sequence.
[31] The probe according to [30], wherein the partial nucleotide sequence p1 at least partially comprises a nucleotide sequence having consecutive 10 or more nucleotides, which is contained in the nucleotide sequence of SEQ ID NO:3, 5, 7, 9 or 11 or a complementary nucleotide sequence thereof.
[32] The probe according to [30] or [31], wherein the partial nucleotide sequence p1 is
   (p1-12-1) a nucleotide sequence having consecutive 10 or more nucleotides and contained in the nucleotide sequence in the range from position 4624 to position 4668 of the nucleotide sequence of SEQ ID NO:2 or a complementary nucleotide sequence thereof,
   (p1-18-1) a nucleotide sequence having consecutive 10 or more nucleotides and contained in the nucleotide sequence in the range from position 2356 to position 2397 of the nucleotide sequence of SEQ ID NO:2 or a complementary nucleotide sequence thereof,
   (p1-20-1) a nucleotide sequence having consecutive 10 or more nucleotides and contained in the nucleotide sequence in the range from position 2624 to position 2663 of the nucleotide sequence of SEQ ID NO:2 or a complementary nucleotide sequence thereof,
   (p1-22-1) a nucleotide sequence having consecutive 10 or more nucleotides and contained in the nucleotide sequence in the range from position 4044 to position 4083 of the nucleotide sequence of SEQ ID NO:2 or a complementary nucleotide sequence thereof,
   (p1-24-1) a nucleotide sequence having consecutive 10 or more nucleotides and contained in the nucleotide sequence in the range from position 6535 to position 6575 of the nucleotide sequence of SEQ ID NO:2 or a complementary nucleotide sequence thereof,
   (p1-26-1) a nucleotide sequence having consecutive 10 or more nucleotides and contained in the nucleotide sequence in the range from position 7478 to position 7520 of the nucleotide sequence of SEQ ID NO:2 or a complementary nucleotide sequence thereof,
   (p1-28-1) a nucleotide sequence having consecutive 10 or more nucleotides and contained in the nucleotide sequence in the range from position 7629 to position 7673 of the nucleotide sequence of SEQ ID NO:2 or a complementary nucleotide sequence thereof,
   (p1-13-1) a nucleotide sequence having consecutive 10 or more nucleotides and contained in the nucleotide sequence in the range from position 4694 to position 4740 of the nucleotide sequence of SEQ ID NO:2 or a complementary nucleotide sequence thereof,
   (p1-19-1) a nucleotide sequence having consecutive 10 or more nucleotides and contained in the nucleotide sequence in the range from position 2672 to position 2711 of the nucleotide sequence of SEQ ID NO:2 or a complementary nucleotide sequence thereof,
   (p1-21-1) a nucleotide sequence having consecutive 10 or more nucleotides and contained in the nucleotide sequence in the range from position 3539 to position 3583 of the nucleotide sequence of SEQ ID NO:2 or a complementary nucleotide sequence thereof,
   (p1-23-1) a nucleotide sequence having consecutive 10 or more nucleotides and contained in the nucleotide sequence in the range from position 5061 to position 5103 of the nucleotide sequence of SEQ ID NO:2 or a complementary nucleotide sequence thereof,
   (p1-25-1) a nucleotide sequence having consecutive 10 or more nucleotides and contained in the nucleotide sequence in the range from position 6566 to position 6605 of the nucleotide sequence of SEQ ID NO:2 or a complementary nucleotide sequence thereof,
   (p1-27-1) a nucleotide sequence having consecutive 10 or more nucleotides and contained in the nucleotide sequence in the range from position 7931 to position 7976 of the nucleotide sequence of SEQ ID NO:2 or a complementary nucleotide sequence thereof, or
   (p1-29-1) a nucleotide sequence having consecutive 10 or more nucleotides and contained in the nucleotide sequence in the range from position 8302 to position 8346 of the nucleotide sequence of SEQ ID NO:2 or a complementary nucleotide sequence thereof.
[33] A method for determining that the base corresponding to position 28 in the nucleotide sequence of SEQ ID NO:1 of erm (41) gene is cytosine, in a mycobacterium, comprising
   performing a nucleic acid amplification reaction using a genomic DNA of the mycobacterium as a subject to be determined or a polynucleotide derived from the genomic DNA of the mycobacterium as a subject to be determined as a template and using the primer set according to any one of [23] to [27],
   detecting an amplified product obtained by the nucleic acid amplification reaction, and
   determining that, when the amplified product is detected, the base in erm (41) gene in the genomic DNA of the mycobacterium is cytosine.
[34] A method for determining sensitivity of a mycobacterium to a macrolide antibiotic substance, comprising the method according to [33], wherein, when the amplified product is detected, the mycobacterium is determined to have a sensitivity to a macrolide antibiotic substance.
[35] A method for determining that the base corresponding to position 28 in the nucleotide sequence of SEQ ID NO:1 of erm (41) gene is cytosine in a mycobacterium, comprising
   incubating a genomic DNA of the mycobacterium as a subject to be determined or a polynucleotide derived from the genomic DNA of the mycobacterium as a subject to be determined, and the probe according to any one of [29] to [32] in a condition allowing hybridization,
   detecting hybridization of the genomic DNA or the polynucleotide and the probe, and
   determining that the base in erm (41) gene of the genomic DNA of the mycobacterium as the subject to be determined is cytosine when the hybridization is detected.
[36] A method for determining sensitivity of a mycobacterium to a macrolide antibiotic substance, comprising the method according to [35], comprising determining that the mycobacterium has a sensitivity to a macrolide antibiotic substance when the hybridization is detected.
[37] Use of the primer set according to any one of [5] to [8], the kit according to [10] or the probe according to any one [11] to [14], for detecting an indicator nucleotide sequence, which is present in the region except erm (41) gene of a genomic DNA when the base corresponding to position 28 in the nucleotide sequence of SEQ ID NO:1 of erm (41) gene is cytosine, and which is not present when the base is thymine, in a mycobacterium.
[38] Use of the primer set according to any one of [5] to [8], the kit according to [10] or the probe according to any one [11] to [14], for determining whether the base corresponding to position 28 in the nucleotide sequence of SEQ ID NO: 1 of erm (41) gene is cytosine or thymine, in a mycobacterium.
[39] Use of the primer set according to any one of [5] to [8], the kit according to [10] or the probe according to any one [11] to [14], for determining sensitivity of a mycobacterium to a macrolide antibiotic substance.
[40] Use of the primer set according to any one of [5] to [8], the kit according to [10] or the probe according to any one [11] to [14], for determining that the base corresponding to position 28 in the nucleotide sequence of SEQ ID NO:1 of erm (41) gene is cytosine, in a mycobacterium.

The specification contains the disclosures of JP Patent Application No. 2020-066277, which serves as a basis for claiming a right of priority of the present application.

### Advantageous Effects of Invention

According to one or more embodiments of the present invention, it is possible to easily determine whether the base at position 28 in erm (41) gene is cytosine or thymine, in a mycobacterium belonging to *Mycobacteroides abscessus* complex. According to one or more embodiments of the present invention, it is also possible to easily determine that the base at position 28 in erm (41) gene is cytosine in a mycobacterium.

### Brief Description of Drawings

[Figure 1] Figure 1 shows a schematic view of a nucleic acid detection device 10. Reference number 1: solid phase support, 2: conjugate pad (label substance holding portion), 3: sample pad (sample receiving portion), 4: absorption pad, 5: substrate, 6: portion having tag capture means.
[Figure 2] Figure 2 shows the detection results of PCR amplified products obtained by using genomic DNA molecules of different mycobacterium strains as templates and primer sets specifically amplifying an indicator nucleotide sequence of SEQ ID NO:2, by nucleic acid chromatography, in Example 2.

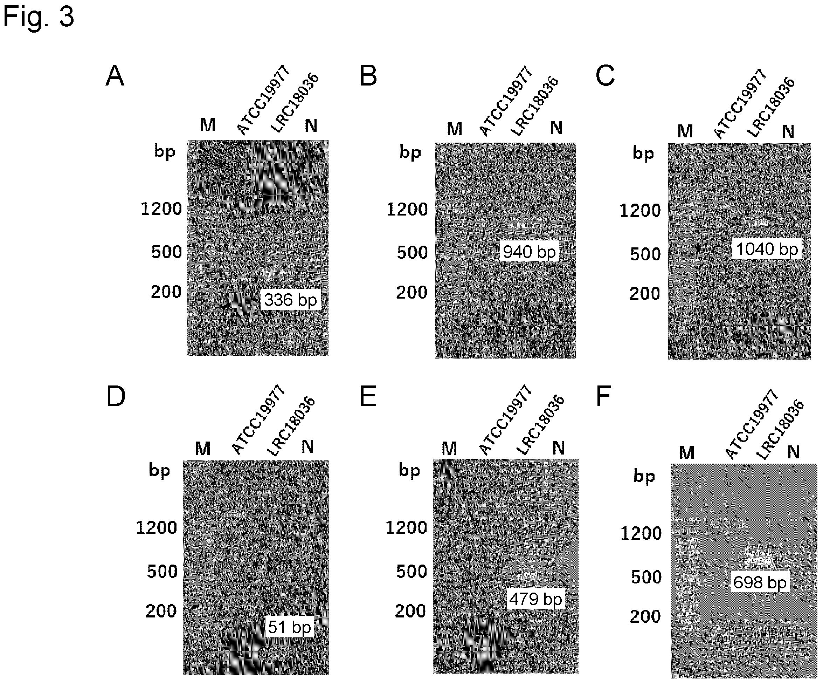

[Figure 3] Figure 3 shows the detection results of amplified products, which are obtained by PCR using genomic DNA molecules of *Mycobacteroides abscessus* ATCC19977 (T28 strain) and *Mycobacteroides abscessus* LRC18036 (C28 strain) as templates and a second to seventh primer sets specifically amplifying different regions of an indicator nucleotide sequence of SEQ ID NO:2, by agarose gel electrophoresis, in Example 2. Figure 3 shows the detection results of PCR amplified products using a second primer set (A), a third primer set (B), a fourth primer set (C), a fifth primer set (D), a sixth primer set (E) and a seventh primer set (F), by agarose gel electrophoresis.

### Description of Embodiments

Hereinafter, the present invention will be described in detail.

### <Terms>

In the present invention, the term such as "a polynucleotide" or "a nucleic acid" refers to a DNA or an RNA, and typically, a DNA. The number of nucleotides of a polynucleotide or a nucleic acid is not particularly limited and an oligonucleotide is included in the term. In the present invention, the polynucleotide or nucleic acid contained in a primer or a probe is typically a polymer formed of naturally occurring nucleotides. The naturally occurring nucleotide refers to a nucleotide constituted of a base being natural adenine, thymine, guanine, cytosine or uracil, a sugar being deoxyribose or ribose, and a phosphate group, in other words, a nucleotide constituted of components having no artificial modification. Naturally occurring nucleotides are usually D-form nucleotides. The D-form nucleotide refers to a nucleotide having a sugar part consisting of D-form deoxyribose or ribose.

The phrase "nucleotide sequence X is capable of hybridizing to nucleotide sequence Y" means that a polynucleotide (particularly, a DNA fragment) containing nucleotide sequence X hybridizes to a polynucleotide (particularly, a DNA fragment) containing nucleotide sequence Y, in a stringent condition, and does not hybridize to a polynucleotide that does not have nucleotide sequence Y. Namely, hybridizing refers to specifically hybridizing. The term "stringent condition" herein refers to a condition under which a so-called specific hybrid is formed and a non-specific hybrid is not formed, and, for example, can be appropriately set with reference to, Green and Sambrook, Molecular Cloning, 4th Ed (2012), Cold Spring Harbor Laboratory Press. Specifically, a stringent condition can be set by a temperature and a salt concentration in a solution during southern hybridization, and a temperature and a salt concentration in a solution during a washing step of southern hybridization. More specifically, examples of the stringent condition include, for example, in a hybridization step, a sodium concentration of 25 to 500 mM, preferably 25 to 300 mM, and a temperature of 40 to 68°C, preferably 40 to 65°C. More specifically, hybridization can be carried out in the conditions: 1 to 7 × SSC (1 × SSC: 150 mM sodium chloride, 15 mM monosodium citrate, pH 7.2), 0.02 to 3% SDS, and a temperature of 40°C to 60°C. After hybridization, a washing step may be carried out, and for example, the washing step may be carried out in the condition: 0.1 to 2 × SSC, 0.1 to 0.3% SDS, and a temperature of 50 to 65°C.

Specific examples of a stringent condition include the following conditions: hybridization of a DNA fragment containing nucleotide sequence X with a DNA fragment containing nucleotide sequence Y is carried out in a hybridization solution containing, for example, 5 × SSC and 0.1% (w/v) N-lauroyl sarcosine, 0.02% (w/v) SDS, overnight (about 8 to 16 hours), and subsequently, washing is performed twice with a washing solution containing 0.1 to 0.5 × SSC, and 0.1% (w/v) SDS, preferably 0.1 × SSC and 0.1% (w/v) SDS, for 15 minutes. A temperature for hybridization and washing is preferably 50°C or more, and more preferably 65°C or more. Preferably, the hybridization solution may further contain a 0.5 to 2 (w/v) blocking reagent for nucleic acid hybridization. After the hybridization and washing in this condition, if the DNA probe containing nucleotide sequence X is hybridized to the DNA fragment containing nucleotide sequence Y, it can be said that nucleotide sequence X is "capable of hybridizing" to nucleotide sequence Y.

In the case where nucleotide sequence X is capable of hybridizing to nucleotide sequence Y, a polynucleotide (particularly a DNA) containing nucleotide sequence X and a polynucleotide (particularly a DNA) containing nucleotide sequence Y may be any combination as long as they hybridize in an annealing condition of a nucleic acid amplification reaction to form hydrogen bonds sufficient for forming a stable double strand. It is not necessary that they are mutually completely complementary nucleotide sequences. For example, mismatches may be present between nucleotide sequence X and nucleotide sequence Y in a ratio of, e.g., one mismatch per 10 nucleotides, one mismatch per 20 nucleotides, or one mismatch per 30nucleotides.

When nucleotide sequence X is capable of hybridizing to nucleotide sequence Y, or when referring to "nucleotide sequence Y capable of hybridizing to nucleotide sequence X", preferably, one or more the following relationships (A) to (C) are satisfied.
(A) Complementary nucleotide sequence of nucleotide sequence X is identical to nucleotide sequence Y. Note that, if one of the complementary nucleotide sequence of nucleotide sequence X and nucleotide sequence Y is a DNA nucleotide sequence and the other one is an RNA nucleotide sequence, thymine of the DNA sequence is regarded as the same base as uracil in the RNA sequence.
(B) Nucleotide sequence Y is a nucleotide sequence in which one or several nucleotides are deleted, substituted, added and/or inserted in the complementary nucleotide sequence of nucleotide sequence X.
(C) Nucleotide sequence Y is a nucleotide sequence having an identity of 80% or more to the complementary nucleotide sequence of nucleotide sequence X.

In the above (B), the term "one or several" refers to preferably 1 to 5, more preferably 1 to 4, more preferably 1 to 3, particularly preferably, 1 or 2, and most preferably, 1.

In the above (C), the value of identity refers to a value calculated in default setting using software (for example, FASTA, DANASYS, and BLAST) for calculating identity between nucleotide sequences. The value of identity of a nucleotide sequence is obtained by aligning a pair of nucleotide sequences such that degree of match is maximum, counting the number of nucleotides matched at that time, and calculating a ratio of the number of matched nucleotides relative to the total number of nucleotides in the nucleotide sequence used in comparison. If there is a gap between the sequences, a single gap is regarded as a single nucleotide in calculating the total number of nucleotides. For details of how to determine identity, see, for example, Altschul et al., Nuc. Acids. Res. 25, 3389-3402, 1977 and Altschul et al., J. Mol. Biol. 215, 403-410, 1990.

In the above (C), the identity is more preferably 90% or more, more preferably 95% or more, more preferably 96% or more, more preferably 97% or more, more preferably 98% or more, and more preferably 99% or more.

Of the above (A) to (C), (A) is particularly preferable.

In the present invention, a method for producing a polynucleotide contained in a primer and/or a probe is not particularly limited. The polynucleotide may be produced by a polynucleotide synthesizer or by an outsourcing synthesis service.

### <Nucleic Acid Amplification Reaction>

When a nucleic acid amplification reaction is performed using a primer set according to one or more embodiments of the present invention, a heat resistant polymerase or a strand-displacement polymerase may be used in the nucleic acid amplification reaction. The polymerase refers to a nucleic acid polymerase such as a DNA polymerase or an RNA polymerase, and preferably, a DNA polymerase.

The nucleic acid amplification reaction using a heat resistant polymerase includes a polymerase chain reaction (PCR). As the heat resistant polymerase, a commercially available DNA polymerase can be used. For example, TaKaRa Ex Taq (registered trademark) can be preferably used. Conditions such as temperature, time and composition of a buffer solution can be appropriately selected depending on e.g., the type of DNA polymerase to be used and concentrations of individual primers. Conditions for PCR such as time, temperature, buffer composition, substrate nucleotide concentration and number of cycles in each of denaturation, annealing and elongation steps of the PCR method can be appropriately set in consideration of, e.g., the DNA polymerase selected, sequences of primers, number of nucleotides of a target nucleic acid and concentration of a template.

The strand-displacement polymerase is an enzyme synthesizing a new DNA strand while self-dissociating hydrogen bonds of a double-stranded nucleic acid containing a target nucleic acid. Examples of the strand-displacement polymerase include φ29 DNA polymerase, Bst DNA polymerase, Klenow fragment of DNA polymerase I, Vent DNA polymerase, Vent (Exo-) DNA polymerase, DeepVent DNA polymerase, DeepVent (Exo-) DNA polymerase, 96-7 DNA polymerase, Aac DNA polymerase and Csa DNA polymerase. When the strand-displacement polymerase is used, a step of dissociating a double strand is not required. Due to this, a nucleic acid can be isothermally amplified.

As the nucleic acid amplification reaction using a strand-displacement polymerase, an isothermal nucleic acid amplification method is preferred. Examples of the isothermal nucleic acid amplification method include RPA method (Recombinase Polymerase Amplification), TRIAmp method (Tandem Repeat-mediated Isothermal Amplification), LAMP method (Loop-mediated isothermal amplification, and HDA method (Helicase-dependent amplification).

The isothermal nucleic acid amplification using a strand-displacement polymerase can be carried out by incubation in the copresence of a template nucleic acid, a primer, a strand-displacement polymerase and substrate nucleotides and at a temperature at which a stable base-pair (bond) can be formed between the template nucleic acid and the amplification primer and enzyme activity can be exerted. Conditions such as temperature, buffer composition, substrate nucleotide concentration and reaction time of the nucleic acid amplification reaction according to isothermal nucleic acid amplification method can be each appropriately set in consideration of, e.g., the strand-displacement polymerase selected, sequences of primers, number of nucleotides of a target nucleic acid and concentration of a template nucleic acid concentration.

The term "target nucleic acid" refers to a nucleic acid comprising a nucleotide sequence to be detected and/or amplified in the indicator nucleotide sequence or a complementary nucleotide sequence to the nucleotide sequence to be detected and/or amplified. A target nucleic acid may be present in the form of a double strand with a complementary nucleic acid thereof. One strand of a target nucleic acid that is present as a double strand may be referred to as a "target nucleic acid". Either a nucleic acid to be detected or a complementary strand thereof may be referred to as the "target nucleic acid". Namely, in the present invention "detecting a target nucleic acid" or "amplifying a target nucleic acid" includes not only detecting or amplifying a target nucleic acid for detecting or amplifying a target nucleic acid itself, but also detecting or amplifying a target nucleic acid, a complementary strand of a target nucleic acid or a double-stranded nucleic acid consisting of a target nucleic acid and a complementary strand thereof for detecting or amplifying a complementary strand of a target nucleic acid or a double-stranded nucleic acid consisting of a target nucleic acid and a complementary strand thereof.

A nucleic acid serving as a template in a nucleic acid amplification reaction may be a DNA or an RNA as long as it contains an indicator nucleotide sequence and/or a complementary nucleotide sequence thereof in part, and is preferably a DNA. Usually, the nucleic acid serving as a template in a nucleic acid amplification reaction is a double strand DNA consisting of a polynucleotide chain containing a target nucleic acid at least in part and a complementary strand of the polynucleotide chain.

A nucleic acid serving as a template may be a naturally occurring nucleic acid or a nucleic acid artificially synthesized. Examples of the nucleic acid include a naturally occurring nucleic acid extracted from a biological sample, a nucleic acid amplified by a nucleic acid amplification reaction such as PCR, and a cDNA synthesized by a reverse transcription reaction.

### <Mycobacterium>

In the specification, the "mycobacterium" as a subject to be determined refers to an anti-acidic and non-motile gram-positive bacillus. In the specification, the "mycobacterium" may include "Nontuberculous mycobacteria; NTM", in particular, "a mycobacterium belonging to "*Mycobacteroides abscessus* complex." *Mycobacteroides abscessus* complex has three subspecies as mentioned above. One or more embodiments of the present invention is preferably used for detecting a mutation in base at position 28 in erm (41) gene in *Mycobacteroides abscessus* complex, and particularly preferably, used for detecting a mutation in base at position 28 in erm (41) gene in *Mycobacteroides abscessus subspecies abscessus* and *Mycobacteroides abscessus subspecies boretti.* Note that, the genus *Mycobacteroides* is sometimes referred to as the genus *Mycobacterium.*

### <erm (41) gene>

The erm (41) gene is a gene encoding erythromycin ribosome methyltransferase. The erm (41) gene expresses erythromycin ribosome methyltransferase in the presence of a macrolide antibiotic substance such as clarithromycin and induces resistance to the macrolide antibiotic substance.

The erm (41) gene contained in a genomic DNA of a standard strain of *Mycobacteroides abscessus* complex has the nucleotide sequence of SEQ ID NO: 1. In the nucleotide sequence of SEQ ID NO: 1, the base at position 28 is thymine. A mycobacterium in which the erm (41) gene has the nucleotide sequence of SEQ ID NO: 1 is a resistant strain that can induce resistance to a macrolide antibiotic substance.

On the other hand, in about 10% of the mycobacteria belonging to *Mycobacteroides abscessus* complex, the nucleotide sequence of erm (41) gene has a mutation from thymine to cytosine at the base corresponding to position 28 of the nucleotide sequence of SEQ ID NO:1. A mycobacterium having such a genotype cannot induce resistance to a macrolide antibiotic substance and is a macrolide antibiotic substance sensitive bacterium.

Note that, some of mycobacteria belonging to *Mycobacteroides abscessus* complex are erm (41) gene partial defective strains, in which erm (41) gene does not have a full length of the nucleotide sequence represented by SEQ ID NO:1, and a part of erm (41) gene is missing. Even in an erm (41) gene partial defective strain, a nucleotide sequence upstream of the base at position 28 in the nucleotide sequence of SEQ ID NO: 1 is present, and a strain in which the base is thymine and a strain in which the base is cytosine are present.

Hereinafter, the type of gene where the base corresponding to position 28 in the nucleotide sequence of SEQ ID NO: 1 of erm (41) gene is thymine will be referred to as "normal type", "erm (41) T28" or "T28". The mycobacterium strain where the base corresponding to position 28 in the nucleotide sequence of SEQ ID NO:1 of erm (41) gene is thymine will be referred to as a "T28 strain". Note that, the nucleotide sequence of erm (41) T28 gene refers to a nucleotide sequence in which a base corresponding to position 28 of the nucleotide sequence of SEQ ID NO:1 is thymine. The nucleotide sequence at other positions may be identical to the nucleotide sequence of SEQ ID NO: 1; may have a deletion, substitution, addition and/or insertion of one or several bases in the nucleotide sequence of SEQ ID NO: 1; or may have a deletion of a partial sequence downstream of position 28 in erm (41) gene, as mentioned above.

Hereinafter, the type of gene in which the base corresponding to position 28 in the nucleotide sequence of SEQ ID NO: 1 of erm (41) gene is cytosine will be referred to as "mutant type", "erm (41) C28" or "C28". The mycobacterium strain in which the base corresponding to position 28 in the nucleotide sequence of SEQ ID NO: 1 of erm (41) gene is cytosine will be referred to as a "C28 strain". Note that, the nucleotide sequence of erm (41) C28 gene refers to a nucleotide sequence in which a base corresponding to position 28 of the nucleotide sequence of SEQ ID NO:1 is cytosine. The nucleotide sequence at other positions may be identical to the nucleotide sequence of SEQ ID NO: 1; may have a deletion, substitution, addition and/or insertion of one or several bases in the nucleotide sequence of SEQ ID NO: 1; or may have a deletion of a partial sequence downstream of position 28 in erm (41) gene, as mentioned above.

In the right above two paragraphs, "one or several" is preferably 1 to 5, more preferably 1 to 4, more preferably 1 to 3, particularly preferably 1 or 2 and most preferably one.

### <Method 1 for Determining Genotype of erm (41) Gene of Mycobacterium>

The first embodiment of the present invention relates to:
a method for determining whether the base corresponding to position 28 in the nucleotide sequence of SEQ ID NO:1 of erm (41) gene is cytosine or thymine in a mycobacterium belonging to *Mycobacteroides abscessus* complex, comprising
detecting, in a genomic DNA of the mycobacterium as a subject to be determined, an indicator nucleotide sequence, which is present in the region except erm (41) gene in the genomic DNA when the base in erm (41) gene is cytosine, and which is not present when the base is thymine, wherein
detection of the indicator nucleotide sequence indicates that the base in erm (41) gene in the genomic DNA of the mycobacterium is cytosine, and
non-detection of the indicator nucleotide sequence indicates that the base in erm (41) gene in the genomic DNA of the mycobacterium is thymine.

According to the method of the first embodiment, it is possible to determine whether a mycobacterium belonging to *Mycobacteroides abscessus* complex is a C28 strain or a 28 strain by specifically detecting an indicator nucleotide sequence present in the region except erm (41) gene of a genomic DNA when the mycobacterium is a C28 strain instead of detecting a single nucleotide mutation in erm (41) gene. In the method according to the first embodiment, it is not necessary to directly detect a single nucleotide mutation, and it is possible to determine whether the mycobacterium is a C28 strain or a T28 strain if the presence or absence of an indicator nucleotide sequence in a genomic DNA can be detected. Therefore, the method can be easily carried out.

The method according to the first embodiment can also be used for determining whether or not a mycobacterium, in particular, a mycobacterium belonging to *Mycobacteroides abscessus* complex is sensitive to a macrolide antibiotic substance. The method for determining sensitivity of a mycobacterium to a macrolide antibiotic substance comprises detecting the indicator nucleotide sequence in a genomic DNA of the mycobacterium as a subject to be determined. In this method, detection of the indicator nucleotide sequence indicates that the mycobacterium has a sensitivity to a macrolide antibiotic substance and non-detection of the indicator nucleotide sequence indicates that the mycobacterium does not have sensitivity to a macrolide antibiotic substance.

The indicator nucleotide sequence can also be used as an indicator for determining whether the base corresponding to position 28 in the nucleotide sequence of SEQ ID NO:1 of erm (41) gene is cytosine in a mycobacterium.

The present inventors surprisingly found that a region having the nucleotide sequence of SEQ ID NO:2 is specifically present in the genomic DNA of a C28 strain and not present in the genomic DNA of a T28 strain.

The nucleotide sequence of SEQ ID NO:2 is a nucleotide sequence in which the nucleotide sequence of SEQ ID NO:3, the nucleotide sequence of SEQ ID NO:4, the nucleotide sequence of SEQ ID NO:5, the nucleotide sequence of SEQ ID NO:6, the nucleotide sequence of SEQ ID NO:7, the nucleotide sequence of SEQ ID NO:8, the nucleotide sequence of SEQ ID NO:9, the nucleotide sequence of SEQ ID NO: 10 and the nucleotide sequence of SEQ ID NO: 11 are linked in the order from the 5' end to the 3' end. The positions of nucleotides at the 5' end and the 3' end of the individual nucleotide sequences of SEQ ID NOT to 11 on the nucleotide sequence of SEQ ID NO:2 are shown in the following table.

**[Table 1]**

| | Position in SEQ ID NO:2 5' end | 3' end | Length (bp) |
|---|---|---|---|
| SEQ ID NOT | 1 | 128 | 128 |
| SEQ ID NO:4 | 129 | 2365 | 2237 |
| SEQ ID NO:5 | 2366 | 3573 | 1208 |
| SEQ ID NO:6 | 3574 | 4053 | 480 |
| SEQ ID NO:7 | 4054 | 5093 | 1040 |
| SEQ ID NO:8 | 5094 | 6431 | 1338 |
| SEQ ID NO:9 | 6432 | 6595 | 164 |
| SEQ ID NO:10 | 6596 | 7487 | 892 |
| SEQ ID NO:11 | 7488 | 8336 | 849 |

In the nucleotide sequence of SEQ ID NO:2, the nucleotide sequence of SEQ ID NO:3, the nucleotide sequence of SEQ ID NO:5, the nucleotide sequence of SEQ ID NO:7, the nucleotide sequence of SEQ ID NO:9 and the nucleotide sequence of SEQ ID NO:11 are particularly preferable as an indicator for determining a C28 strain, since there are no nucleotide sequences having relatively high homology to these sequences in the genomic DNA of a T28 strain.

The indicator nucleotide sequence may be any nucleotide sequence as long as it is specifically present in the genomic DNA of a mycobacterium C28 strain. More specifically, as the indicator nucleotide sequence, a first nucleotide sequence having consecutive 10 or more nucleotides contained in the nucleotide sequence of SEQ ID NO:2, or a second nucleotide sequence complementary to the first nucleotide sequence, is preferable. The first nucleotide sequence is more preferably a nucleotide sequence having consecutive 20 or more nucleotides, 50 or more nucleotides, 100 or more nucleotides, 500 or more nucleotides, or 1000 or more nucleotides contained in the nucleotide sequence of SEQ ID NO:2. The upper limit of the number of nucleotides in the first nucleotide sequence is not particularly limited but it may be, for example, 3000 or less nucleotides, or 1500 or less nucleotides.

The first nucleotide sequence at least partially contains a nucleotide sequence of the following: more preferably consecutive 10 or more nucleotides, preferably 20 or more nucleotides, 50 or more nucleotides, 100 or more nucleotides, 500 or more nucleotides, or 1000 or more nucleotides contained in the nucleotide sequence of SEQ ID NO:3, 5, 7, 9 or 11. The first nucleotide sequence or its complementary nucleotide sequence, i.e., the second nucleotide sequence, is a nucleotide sequence to which no relatively high homologous sequence is present in the genomic DNA of a T28 strain. Thus, based on the presence of the nucleotide sequence, a C28 strain can be highly accurately determined.

In the method according to the first embodiment, although a method for detecting the indicator nucleotide sequence is not particularly limited, and, for example, a method using a probe or a primer can be employed for the detection.

In the method according to the first embodiment, if the indicator nucleotide sequence is detected in a genomic DNA of a mycobacterium belonging to *Mycobacteroides abscessus* complex, the mycobacterium can be determined as a C28 strain, and if the indicator nucleotide sequence is not detected in a genomic DNA of a mycobacterium, the mycobacterium can be determined as a T28 strain.

### <Probe for Detecting Indicator Nucleotide Sequence Specific to C28 Strain>

A second embodiment of the present invention relates to the following.

A probe for detecting an indicator nucleotide sequence, which is present in the region except erm (41) gene of a genomic DNA when the base corresponding to position 28 in the nucleotide sequence of SEQ ID NO:1 of erm (41) gene is cytosine and which is not present when the base is thymine, in a mycobacterium belonging to *Mycobacteroides abscessus* complex,
the probe comprising a polynucleotide comprising a nucleotide sequence capable of hybridizing to a partial nucleotide sequence p1 having consecutive 10 or more nucleotides and contained in the indicator nucleotide sequence.

In the second embodiment, the indicator nucleotide sequence can be selected from the same range as that of the indicator nucleotide sequence according to the first embodiment.

The partial nucleotide sequence p1 is present in the region of the indicator nucleotide sequence hybridizing to the probe according to the second embodiment.

The partial nucleotide sequence p1 is a continuous partial nucleotide sequence in the indicator nucleotide sequence. The partial nucleotide sequence p1 is satisfactory as long as it has the number of nucleotides required for maintaining specificity. The number of nucleotides is satisfactorily 10 or more nucleotides, more preferably 15 or more nucleotides, more preferably 17 or more nucleotides, and more preferably 20 or more nucleotides. The upper limit of the number of nucleotides of the partial nucleotide sequence p1 is not particularly limited but the partial nucleotide sequence p1 may be a partial nucleotide sequence of the indicator nucleotide sequence having, for example, 400 or less nucleotides, 300 or less nucleotides, 200 or less nucleotides, 100 or less nucleotides, or 50 or less nucleotides.

More preferably, the partial nucleotide sequence p1 at least partially contains a nucleotide sequence having consecutive 10 or more nucleotides, more preferably 15 or more nucleotides, more preferably 17 or more nucleotides, more preferably 20 or more nucleotides, and for example 400 or less nucleotides, 300 or less nucleotides, 200 or less nucleotides, 100 or less nucleotides, or 50 or less nucleotides contained in a nucleotide sequence of SEQ ID NO:3, 5, 7, 9 or 11 or a complementary nucleotide sequence thereof.

The partial nucleotide sequence p1 is preferably
(p1-12-1) a nucleotide sequence having consecutive 10 or more nucleotides, more preferably 17 or more nucleotides, and more preferably 20 or more nucleotides contained in the nucleotide sequence in the range from position 4624 to position 4668 in the nucleotide sequence of SEQ ID NO:2 or a complementary nucleotide sequence thereof,
(p1-18-1) a nucleotide sequence having consecutive 10 or more nucleotides, more preferably 17 or more nucleotides, and more preferably 20 or more nucleotides, contained in the nucleotide sequence in the range from position 2356 to position 2397 in the nucleotide sequence of SEQ ID NO:2 or a complementary nucleotide sequence thereof,
(p1-20-1) a nucleotide sequence having consecutive 10 or more nucleotides, more preferably 17 or more nucleotides, and more preferably 20 or more nucleotides contained in the nucleotide sequence in the range from position 2624 to position 2663 in the nucleotide sequence of SEQ ID NO:2 or a complementary nucleotide sequence thereof,
(p1-22-1) a nucleotide sequence having consecutive 10 or more nucleotides, more preferably 17 or more nucleotides, and more preferably 20 or more nucleotides contained in the nucleotide sequence in the range from position 4044 to position 4083 in the nucleotide sequence of SEQ ID NO:2 or a complementary nucleotide sequence thereof,
(p1-24-1) a nucleotide sequence having consecutive 10 or more nucleotides, more preferably 17 or more nucleotides, and more preferably 20 or more nucleotides contained in the nucleotide sequence in the range from position 6535 to position 6575 in the nucleotide sequence of SEQ ID NO:2 or a complementary nucleotide sequence thereof,
(p1-26-1) a nucleotide sequence having consecutive 10 or more nucleotides, more preferably 17 or more nucleotides, and more preferably 20 or more nucleotides contained in the nucleotide sequence in the range from position 7478 to position 7520 in the nucleotide sequence of SEQ ID NO:2 or a complementary nucleotide sequence thereof,
(p1-28-1) a nucleotide sequence having consecutive 10 or more nucleotides, more preferably 17 or more nucleotides, and more preferably 20 or more nucleotides contained in the nucleotide sequence in the range from position 7629 to position 7673 in the nucleotide sequence of SEQ ID NO:2 or a complementary nucleotide sequence thereof,
(p1-13-1) a nucleotide sequence having consecutive 10 or more nucleotides, more preferably 17 or more nucleotides, and more preferably 20 or more nucleotides contained in the nucleotide sequence in the range from position 4694 to position 4740 in the nucleotide sequence of SEQ ID NO:2 or a complementary nucleotide sequence thereof,
(p1-19-1) a nucleotide sequence having consecutive 10 or more nucleotides, more preferably 17 or more nucleotides, and more preferably 20 or more nucleotides contained in the nucleotide sequence in the range from position 2672 to position 2711 in the nucleotide sequence of SEQ ID NO:2 or a complementary nucleotide sequence thereof,
(p1-21-1) a nucleotide sequence having consecutive 10 or more nucleotides, more preferably 17 or more nucleotides, and more preferably 20 or more nucleotides contained in the nucleotide sequence in the range from position 3539 to position 3583 in the nucleotide sequence of SEQ ID NO:2 or a complementary nucleotide sequence thereof,
(p1-23-1) a nucleotide sequence having consecutive 10 or more nucleotides, more preferably 17 or more nucleotides, and more preferably 20 or more nucleotides contained in the nucleotide sequence in the range from position 5061 to position 5103 in the nucleotide sequence of SEQ ID NO:2 or a complementary nucleotide sequence thereof,
(p1-25-1) a nucleotide sequence having consecutive 10 or more nucleotides, more preferably 17 or more nucleotides, and more preferably 20 or more nucleotides contained in the nucleotide sequence in the range from position 6566 to position 6605 in the nucleotide sequence of SEQ ID NO:2 or a complementary nucleotide sequence thereof,
(p1-27-1) a nucleotide sequence having consecutive 10 or more nucleotides, more preferably 17 or more nucleotides, and more preferably 20 or more nucleotides contained in the nucleotide sequence in the range from position 7931 to position 7976 in the nucleotide sequence of SEQ ID NO:2 or a complementary nucleotide sequence thereof, or
(p1-29-1) a nucleotide sequence having consecutive 10 or more nucleotides, more preferably 17 or more nucleotides, and more preferably 20 or more nucleotides contained in the nucleotide sequence in the range from position 8302 to position 8346 in the nucleotide sequence of SEQ ID NO:2 or a complementary nucleotide sequence thereof; and more preferably,
(p1-12-2) a nucleotide sequence having consecutive 10 or more nucleotides, more preferably 17 or more nucleotides, and more preferably 20 or more nucleotides contained in the nucleotide sequence in the range from position 4629 to position 4663 in the nucleotide sequence of SEQ ID NO:2 or a complementary nucleotide sequence thereof,
(p1-18-2) a nucleotide sequence having consecutive 10 or more nucleotides, more preferably 17 or more nucleotides, and more preferably 20 or more nucleotides contained in the nucleotide sequence in the range from position 2361 to position 2392 in the nucleotide sequence of SEQ ID NO:2 or a complementary nucleotide sequence thereof,
(p1-20-2) a nucleotide sequence having consecutive 10 or more nucleotides, more preferably 17 or more nucleotides, and more preferably 20 or more nucleotides contained in the nucleotide sequence in the range from position 2629 to position 2658 in the nucleotide sequence of SEQ ID NO:2 or a complementary nucleotide sequence thereof,
(p1-22-2) a nucleotide sequence having consecutive 10 or more nucleotides, more preferably 17 or more nucleotides, and more preferably 20 or more nucleotides contained in the nucleotide sequence in the range from position 4049 to position 4078 in the nucleotide sequence of SEQ ID NO:2 or a complementary nucleotide sequence thereof,
(p1-24-2) a nucleotide sequence having consecutive 10 or more nucleotides, more preferably 17 or more nucleotides, and more preferably 20 or more nucleotides contained in the nucleotide sequence in the range from position 6540 to position 6570 in the nucleotide sequence of SEQ ID NO:2 or a complementary nucleotide sequence thereof,
(p1-26-2) a nucleotide sequence having consecutive 10 or more nucleotides, more preferably 17 or more nucleotides, and more preferably 20 or more nucleotides contained in the nucleotide sequence in the range from position 7483 to position 7515 in the nucleotide sequence of SEQ ID NO:2 or a complementary nucleotide sequence thereof,
(p1-28-2) a nucleotide sequence having consecutive 10 or more nucleotides, more preferably 17 or more nucleotides, and more preferably 20 or more nucleotides contained in the nucleotide sequence in the range from position 7634 to position 7668 in the nucleotide sequence of SEQ ID NO:2 or a complementary nucleotide sequence thereof,
(p1-13-2) a nucleotide sequence having consecutive 10 or more nucleotides, more preferably 17 or more nucleotides, and more preferably 20 or more nucleotides contained in the nucleotide sequence in the range from position 4699 to position 4735 in the nucleotide sequence of SEQ ID NO:2 or a complementary nucleotide sequence thereof,
(p1-19-2) a nucleotide sequence having consecutive 10 or more nucleotides, more preferably 17 or more nucleotides, and more preferably 20 or more nucleotides contained in the nucleotide sequence in the range from position 2677 to position 2706 in the nucleotide sequence of SEQ ID NO:2 or a complementary nucleotide sequence thereof,
(p1-21-2) a nucleotide sequence having consecutive 10 or more nucleotides, more preferably 17 or more nucleotides, and more preferably 20 or more nucleotides contained in the nucleotide sequence in the range from position 3544 to position 3578 in the nucleotide sequence of SEQ ID NO:2 or a complementary nucleotide sequence thereof,
(p1-23-2) a nucleotide sequence having consecutive 10 or more nucleotides, more preferably 17 or more nucleotides, and more preferably 20 or more nucleotides contained in the nucleotide sequence in the range from position 5066 to position 5098 in the nucleotide sequence of SEQ ID NO:2 or a complementary nucleotide sequence thereof,
(p1-25-2) a nucleotide sequence having consecutive 10 or more nucleotides, more preferably 17 or more nucleotides, and more preferably 20 or more nucleotides contained in the nucleotide sequence in the range from position 6571 to position 6600 in the nucleotide sequence of SEQ ID NO:2 or a complementary nucleotide sequence thereof,
(p1-27-2) a nucleotide sequence having consecutive 10 or more nucleotides, more preferably 17 or more nucleotides, and more preferably 20 or more nucleotides contained in the nucleotide sequence in the range from position 7936 to position 7971 in the nucleotide sequence of SEQ ID NO:2 or a complementary nucleotide sequence thereof, or
(p1-29-2) a nucleotide sequence having consecutive 10 or more nucleotides, more preferably 17 or more nucleotides, and more preferably 20 or more nucleotides contained in the nucleotide sequence in the range from position 8307 to position 8341 in the nucleotide sequence of SEQ ID NO:2 or a complementary nucleotide sequence thereof.

The probe according to the second embodiment of the present invention can hybridize to the region of the partial nucleotide sequence p1 of the polynucleotide containing the indicator nucleotide sequence, in a stringent condition. Because of this, the probe according to the second embodiment of the present invention can be used in a nucleic acid hybridization method (for example, Southern hybridization) and real time PCR method (for example, TaqManTM method, Molecular Beacon method).

A polynucleotide constituting the probe according to the second embodiment contains the identical or homologous nucleotide sequence to a complementary nucleotide sequence to the partial nucleotide sequence p1. The "homologous" herein means satisfying the condition of (B) or (C) mentioned above, more specifically, the following condition (B1) or (C1).

(B1) The nucleotide sequence of a polynucleotide constituting the probe according to the second embodiment is a nucleotide sequence in which one or several nucleotides are deleted, substituted, added and/or inserted in a complementary nucleotide sequence to the partial nucleotide sequence p1.

(C1) The nucleotide sequence of a polynucleotide constituting the probe according to the second embodiment is a nucleotide sequence having an identity of 80% or more to a complementary nucleotide sequence to the partial nucleotide sequence p1.

The preferable range of "one or several" in the condition (B1) and "identity" in the condition (C1) are as described above in connection with conditions (B) and (C), respectively.

A polynucleotide constituting the probe according to the second embodiment needs not consist only of the identical or homologous nucleotide sequence to a complementary nucleotide sequence (hereinafter referred to as "nucleotide sequence q1") of the partial nucleotide sequence p1. Other nucleotide sequence(s) may be added to one or both of the 5' end and the 3' end sides of nucleotide sequence q1. Nucleotide sequence q1 is particularly preferably identical to a complementary nucleotide sequence to the partial nucleotide sequence p1.

Although the total number of nucleotides of a polynucleotide constituting the probe according to the second embodiment is not particularly limited, the total number can be 8 or more nucleotides, 10 or more nucleotides, more preferably, 15 or more nucleotides, and more preferably 20 or more nucleotides; and preferably 400 or less nucleotides, 300 or less nucleotides, or 200 or less nucleotides. If the probe is used in real time PCR method, polynucleotide preferably has 50 or less nucleotides, and more preferably 40 or less nucleotides, or 35 or less nucleotides.

Examples of the polynucleotide constituting the probe according to the second embodiment include a polynucleotide containing consecutive 10 or more nucleotides, more preferably 15 or more nucleotides, more preferably 17 or more nucleotides, and particularly preferably 20 or more nucleotides contained in a nucleotide sequence of any one of SEQ ID NO: 12, 13, and 18 to 29 or a complementary nucleotide sequence thereof.

The probe according to the second embodiment may be prepared by further adding a label portion that is a tag capable of binding to a label substance or a label substance, to the polynucleotide. Examples of the label are as described later in connection with the primer. A complex produced by hybridizing a probe containing a label to the polynucleotide containing the indicator nucleotide sequence, can be easily detected using the label as an indicator.

The probe according to the second embodiment may be obtained by further adding a binding portion that is a tag capable of binding to a solid phase support, to the polynucleotide. Examples of the binding portion are as described later in connection with a primer. The complex formed by hybridizing the probe containing a binding portion to a polynucleotide containing the indicator nucleotide sequence can be fixed to a solid phase support via the binding portion. Because of this, the complex can be easily detected and isolated.

The probe containing a binding portion may be previously fixed to a solid phase support via the binding portion and then provided. The probe fixed to a solid phase support can capture a polynucleotide containing the indicator nucleotide sequence.

When the probe according to the second embodiment is used in a real time PCR method, it is preferable to label the probe with a label substance usually used for real-time detection of an amplified product. For example, a reporter fluorescent substance is linked to the 5' end of a polynucleotide to be used as a probe and a quencher dye is linked to the 3' end thereof. Examples of the reporter fluorescent substance include carboxyfluorescein (FAM), hexachlorofluorescein (HEX) and tetrachlorofluorescein (TET). Examples of the quencher dye include fluorescent substances such as carboxytetramethylrhodamine (TAMRA) and non-fluorescent substances such as Black Hole Quencher dye (BHQ) and 4-((4-(dimethylamino)phenyl)azo)benzoic acid (DABCYL).

The probe according to the second embodiment can be used in a method for determining whether the base corresponding to position 28 in the nucleotide sequence of SEQ ID NO:1 of erm (41) gene is cytosine or thymine, in a mycobacterium belonging to *Mycobacteroides abscessus* complex.

To describe more specifically, the method can comprises
incubating a genomic DNA of a mycobacterium as a subject to be determined or a polynucleotide derived from the genomic DNA of the mycobacterium as a subject to be determined, and the probe in a condition allowing hybridization,
detecting hybridization of the genomic DNA or the polynucleotide and the probe, and
determining that the base in erm (41) gene in the genomic DNA of the mycobacterium as the subject to be determined is cytosine when the hybridization is detected, and that the base is thymine when the hybridization is not detected.

The probe according to the second embodiment can be used in a method for determining sensitivity of a mycobacterium, in particular, a mycobacterium belonging to *Mycobacteroides abscessus* complex, to macrolide antibiotic substance. To describe more specifically, the method can comprises
incubating a genomic DNA of a mycobacterium as a subject to be determined or a polynucleotide derived from the genomic DNA of the mycobacterium as a subject to be determined and the probe in a condition allowing hybridization,
detecting hybridization of the genomic DNA or the polynucleotide and the probe, and
determining that the mycobacterium has a sensitivity to a macrolide antibiotic substance when the hybridization is detected; and that the mycobacterium does not have a sensitivity to a macrolide antibiotic substance when the hybridization is not detected.

The probe according to the second embodiment can be used in a method for determining that the base corresponding to position 28 in the nucleotide sequence of SEQ ID NO:1 of erm (41) gene is cytosine in a mycobacterium. To describe more specifically, the method can comprises
incubating a genomic DNA of a mycobacterium as a subject to be determined or a polynucleotide derived from the genomic DNA of the mycobacterium as a subject to be determined and the probe in a condition allowing hybridization,
detecting hybridization of the genomic DNA or the polynucleotide and the probe, and
determining that the base corresponding to the base in erm (41) gene in the genomic DNA of the mycobacterium as the subject to be determined is cytosine when the hybridization is detected.

Examples of the "polynucleotide derived from a genomic DNA of a mycobacterium as a subject to be determined" herein include a partial polynucleotide, which is part of the genomic DNA, and an amplified product obtained by amplifying a whole or part of the genomic DNA containing the indicator nucleotide sequence by a nucleic acid amplification reaction.

In the above embodiments, the "condition allowing hybridization" is preferably the same condition as that specified as the "stringent condition".

### <Primer set for detecting indicator nucleotide sequence specific to C28 strain>

A third embodiment of the present invention relates to a primer set for detecting an indicator nucleotide sequence, which is present in the region except erm (41) gene of a genomic DNA when the base corresponding to position 28 in the nucleotide sequence of SEQ ID NO: 1 of erm (41) gene is cytosine and which is not present when the base is thymine, in a mycobacterium belonging to *Mycobacteroides abscessus* complex, the primer set comprising:
a first primer comprising a polynucleotide comprising, at the 3' end, a nucleotide sequence f12 capable of hybridizing to a complementary nucleotide sequence to a partial nucleotide sequence f11 having consecutive 10 or more nucleotides which is contained in the indicator nucleotide sequence, and
a second primer comprising a polynucleotide comprising, at the 3' end, a nucleotide sequence r12 capable of hybridizing to a partial nucleotide sequence r11 having consecutive 10 or more nucleotides which is contained in the indicator nucleotide sequence, and positioned closer to the 3' end than the 3' end of the partial nucleotide sequence f11.

As the indicator nucleotide sequence, a nucleotide sequence specifically present in the genomic DNA of a mycobacterium C28 strain is satisfactory, and more specifically, a third nucleotide sequence contained in the nucleotide sequence of SEQ ID NO: 2 and having consecutive 20 or more nucleotides is preferred. The third nucleotide sequence is more preferably a nucleotide sequence having consecutive 40 or more nucleotides, 50 or more nucleotides, 100 or more nucleotides, 500 or more nucleotides, or 1000 or more nucleotides contained in the nucleotide sequence of SEQ ID NO:2. The upper limit of number of nucleotides in the third nucleotide sequence is not particularly limited but it may be, for example, 3000 or less nucleotides, or 1500 or less nucleotides.

A nucleotide sequence from the nucleotide at the 5' end of the partial nucleotide sequence f11 to the nucleotide at the 3' end of the partial nucleotide sequence r11 contained in the third nucleotide sequence will be hereinafter referred to as a "target sequence". The target sequence and a complementary nucleotide sequence thereof are amplified by a nucleic acid amplification reaction using a primer set according to the embodiment. The target sequence at least partially contains preferably a nucleotide sequence having consecutive 20 or more nucleotides, preferably 40 or more nucleotides, 50 or more nucleotides, 100 or more nucleotides, 500 or more nucleotides, or 1000 or more nucleotides and contained in the nucleotide sequence of SEQ ID NO:3, 5, 7, 9 or 11. Since a nucleotide sequence having a relatively high homology to the target sequence is not present in the genomic DNA of a T28 strain, a strain can be highly accurately determined as a C28 strain based on the presence of the target sequence.

The length of the target sequence is not particularly limited. To enhance detection accuracy, it is preferable that the target sequence preferably contains a part consisting of consecutive 20 or more nucleotides, more preferably 40 or more nucleotides, 50 or more nucleotides, 100 or more nucleotides, 500 or more nucleotides, or 1000 or more nucleotides, of the indicator nucleotide sequence. The upper limit of the length of the target sequence is not particularly limited but it is usually 3000 or less nucleotides, or 1500 or less nucleotides.

The partial nucleotide sequence f11 is located at the 5' end of a target sequence. The partial nucleotide sequence r11 is located at the 3' end of the target sequence.

The lengths of the partial nucleotide sequence f11 and the partial nucleotide sequence r11 may be each 10 or more nucleotides, more preferably 15 or more nucleotides, more preferably 17 or more nucleotides, and more preferably 20 or more nucleotides, and typically 40 or less nucleotides, 30 or less nucleotides, or 27 or less nucleotides.

The partial nucleotide sequence f11 is preferably,
(f11-12-1) a nucleotide sequence having consecutive 10 or more nucleotides, more preferably 17 or more nucleotides, more preferably 20 or more nucleotides, contained in the nucleotide sequence in the range from position 4624 to position 4668 of the nucleotide sequence of SEQ ID NO:2
(f11-18-1) a nucleotide sequence having consecutive 10 or more nucleotides, more preferably 17 or more nucleotides, and more preferably 20 or more nucleotides, contained in the nucleotide sequence in the range from position 2356 to position 2397 of the nucleotide sequence of SEQ ID NO:2,
(f11-20-1) a nucleotide sequence having consecutive 10 or more nucleotides, more preferably 17 or more nucleotides, and more preferably 20 or more nucleotides, contained in the nucleotide sequence in the range from position 2624 to position 2663 of the nucleotide sequence of SEQ ID NO:2,
(f11-22-1) a nucleotide sequence having consecutive 10 or more nucleotides, more preferably 17 or more nucleotides, and more preferably 20 or more nucleotides, contained in the nucleotide sequence in the range from position 4044 to position 4083 of the nucleotide sequence of SEQ ID NO:2,
(f11-24-1) a nucleotide sequence having consecutive 10 or more nucleotides, more preferably 17 or more nucleotides, and more preferably 20 or more nucleotides, contained in the nucleotide sequence in the range from position 6535 to position 6575 of the nucleotide sequence of SEQ ID NO:2,
(f11-26-1) a nucleotide sequence having consecutive 10 or more nucleotides, more preferably 17 or more nucleotides, and more preferably 20 or more nucleotides, contained in the nucleotide sequence in the range from position 7478 to position 7520 of the nucleotide sequence of SEQ ID NO:2, or
(f11-28-1) a nucleotide sequence having consecutive 10 or more nucleotides, more preferably 17 or more nucleotides, and more preferably 20 or more nucleotides, contained in the nucleotide sequence in the range from position 7629 to position 7673 of the nucleotide sequence of SEQ ID NO:2,
more preferably,
(f11-12-2) a nucleotide sequence having consecutive 10 or more nucleotides, more preferably 17 or more nucleotides, and more preferably 20 or more nucleotides, contained in the nucleotide sequence in the range from position 4629 to position 4661 in the nucleotide sequence of SEQ ID NO:2,
(f11-18-2) a nucleotide sequence having consecutive 10 or more nucleotides, more preferably 17 or more nucleotides, and more preferably 20 or more nucleotides, contained in the nucleotide sequence in the range from position 2361 to position 2390 of the nucleotide sequence of SEQ ID NO:2,
(f1 1-20-2) a nucleotide sequence having consecutive 10 or more nucleotides, more preferably 17 or more nucleotides, and more preferably 20 or more nucleotides, contained in the nucleotide sequence in the range from position 2629 to position 2656 in the nucleotide sequence of SEQ ID NO:2,
(f1 1-22-2) a nucleotide sequence having consecutive 10 or more nucleotides, more preferably 17 or more nucleotides, and more preferably 20 or more nucleotides, contained in the nucleotide sequence in the range from position 4049 to position 4076 in the nucleotide sequence of SEQ ID NO:2,
(f11-24-2) a nucleotide sequence having consecutive 10 or more nucleotides, more preferably 17 or more nucleotides, and more preferably 20 or more nucleotides, contained in the nucleotide sequence in the range from position 6540 to position 6568 in the nucleotide sequence of SEQ ID NO:2,
(f11-26-2) a nucleotide sequence having consecutive 10 or more nucleotides, more preferably 17 or more nucleotides, and more preferably 20 or more nucleotides, contained in the nucleotide sequence in the range from position 7483 to position 7513 in the nucleotide sequence of SEQ ID NO:2, or
(f1 1-28-2) a nucleotide sequence having consecutive 10 or more nucleotides, more preferably 17 or more nucleotides, and more preferably 20 or more nucleotides, contained in the nucleotide sequence in the range from position 7634 to position 7666 in the nucleotide sequence of SEQ ID NO:2, and
particularly preferably,
(f11-12-3) a nucleotide sequence having consecutive 10 or more nucleotides, more preferably 17 or more nucleotides, and more preferably 20 or more nucleotides, contained in the nucleotide sequence in the range from position 4629 to position 4658 in the nucleotide sequence of SEQ ID NO:2,
(f11-18-3) a nucleotide sequence having consecutive 10 or more nucleotides, more preferably 17 or more nucleotides, and more preferably 20 or more nucleotides, contained in the nucleotide sequence in the range from position 2361 to position 2387 in the nucleotide sequence of SEQ ID NO:2,
(f1 1-20-3) a nucleotide sequence having consecutive 10 or more nucleotides, more preferably 17 or more nucleotides, and more preferably 20 or more nucleotides, contained in the nucleotide sequence in the range from position 2629 to position 2653 in the nucleotide sequence of SEQ ID NO:2
(f1 1-22-3) a nucleotide sequence having consecutive 10 or more nucleotides, more preferably 17 or more nucleotides, and more preferably 20 or more nucleotides, contained in the nucleotide sequence in the range from position 4049 to position 4073 in the nucleotide sequence of SEQ ID NO:2,
(f11-24-3) a nucleotide sequence having consecutive 10 or more nucleotides, more preferably 17 or more nucleotides, and more preferably 20 or more nucleotides, contained in the nucleotide sequence in the range from position 6540 to position 6565 in the nucleotide sequence of SEQ ID NO:2,
(f11-26-3) a nucleotide sequence having consecutive 10 or more nucleotides, more preferably 17 or more nucleotides, and more preferably 20 or more nucleotides, contained in the nucleotide sequence in the range from position 7483 to position 7510 in the nucleotide sequence of SEQ ID NO:2, or
(f1 1-28-3) a nucleotide sequence having consecutive 10 or more nucleotides, more preferably 17 or more nucleotides, and more preferably 20 or more nucleotides, contained in the nucleotide sequence in the range from position 7634 to position 7663 in the nucleotide sequence of SEQ ID NO:2.

In nucleotide sequences (f11-12-1) to (f11-28-1), the "nucleotide sequence having consecutive 10 or more nucleotides, more preferably 17 or more nucleotides, and more preferably 20 or more nucleotides" is preferably a "nucleotide sequence having consecutive 10 or more nucleotides, more preferably 17 or more nucleotides, and more preferably 20 or more nucleotides starting from any of the 1 to 12th nucleotide counted from the 3' end, toward the 5' end".

In nucleotide sequences (f11-12-2) to (f11-28-2), the "nucleotide sequence having consecutive 10 or more nucleotides, more preferably 17 or more nucleotides, and more preferably 20 or more nucleotides" is preferably a "nucleotide sequence having consecutive 10 or more nucleotides, more preferably 17 or more nucleotides, and more preferably 20 or more nucleotides starting from any of the 1 to 5th nucleotide counted from the 3' end, toward the 5' end".

In the sequences (f11-12-3) to (f11-28-3) mentioned above, the "nucleotide sequence having consecutive 10 or more nucleotides, more preferably 17 or more nucleotides, and more preferably 20 or more nucleotides" is preferably a "nucleotide sequence having consecutive 10 or more nucleotides, more preferably 17 or more nucleotides, and more preferably 20 or more nucleotides starting from any of 1 or 2nd nucleotide counted from the 3' end, toward the 5' end. The "nucleotide at any of 1 or 2nd nucleotide counted from the 3' end" is more preferably "the nucleotide at the 3' end".

The partial nucleotide sequence r11 is preferably,
(r11-13-1) a nucleotide sequence having consecutive 10 or more nucleotides, more preferably 17 or more nucleotides, and more preferably 20 or more nucleotides contained in the nucleotide sequence in the range from position 4694 to position 4740 of the nucleotide sequence of SEQ ID NO:2,
(r11-19-1) a nucleotide sequence having consecutive 10 or more nucleotides, more preferably 17 or more nucleotides, and more preferably 20 or more nucleotides contained in the nucleotide sequence in the range from position 2672 to position 2711 of the nucleotide sequence of SEQ ID NO:2,
(r11-21-1) a nucleotide sequence having consecutive 10 or more nucleotides, more preferably 17 or more nucleotides, and more preferably 20 or more nucleotides contained in the nucleotide sequence in the range from position 3539 to position 3583 of the nucleotide sequence of SEQ ID NO:2,
(r11-23-1) a nucleotide sequence having consecutive 10 or more nucleotides, more preferably 17 or more nucleotides, and more preferably 20 or more nucleotides contained in the nucleotide sequence in the range from position 5061 to position 5103 of the nucleotide sequence of SEQ ID NO:2,
(r11-25-1) a nucleotide sequence having consecutive 10 or more nucleotides, more preferably 17 or more nucleotides, and more preferably 20 or more nucleotides contained in the nucleotide sequence in the range from position 6566 to position 6605 of the nucleotide sequence of SEQ ID NO:2,
(r11-27-1) a nucleotide sequence having consecutive 10 or more nucleotides, more preferably 17 or more nucleotides, and more preferably 20 or more nucleotides contained in the nucleotide sequence in the range from position 7931 to position 7976 of the nucleotide sequence of SEQ ID NO:2, or
(r11-29-1) a nucleotide sequence having consecutive 10 or more nucleotides, more preferably 17 or more nucleotides, and more preferably 20 or more nucleotides contained in the nucleotide sequence in the range from position 8302 to position 8346 of the nucleotide sequence of SEQ ID NO:2,
more preferably,
(r11-13-2) a nucleotide sequence having consecutive 10 or more nucleotides, more preferably 17 or more nucleotides, and more preferably 20 or more nucleotides contained in the nucleotide sequence in the range from position 4701 to position 4735 in the nucleotide sequence of SEQ ID NO:2,
(r11-19-2) a nucleotide sequence having consecutive 10 or more nucleotides, more preferably 17 or more nucleotides, and more preferably 20 or more nucleotides contained in the nucleotide sequence in the range from position 2679 to position 2706 in the nucleotide sequence of SEQ ID NO:2,
(r11-21-2) a nucleotide sequence having consecutive 10 or more nucleotides, more preferably 17 or more nucleotides, and more preferably 20 or more nucleotides contained in the nucleotide sequence in the range from position 3546 to position 3578 in the nucleotide sequence of SEQ ID NO:2,
(r11-23-2) a nucleotide sequence having consecutive 10 or more nucleotides, more preferably 17 or more nucleotides, and more preferably 20 or more nucleotides contained in the nucleotide sequence in the range from position 5068 to position 5098 in the nucleotide sequence of SEQ ID NO:2,
(r 11-25-2) a nucleotide sequence having consecutive 10 or more nucleotides, more preferably 17 or more nucleotides, and more preferably 20 or more nucleotides contained in the nucleotide sequence in the range from position 6573 to position 6600 in the nucleotide sequence of SEQ ID NO:2,
(r 11-27-2) a nucleotide sequence having consecutive 10 or more nucleotides, more preferably 17 or more nucleotides, and more preferably 20 or more nucleotides contained in the nucleotide sequence in the range from position 7938 to position 7971 in the nucleotide sequence of SEQ ID NO:2, or
(r 11-29-2) a nucleotide sequence having consecutive 10 or more nucleotides, more preferably 17 or more nucleotides, and more preferably 20 or more nucleotides contained in the nucleotide sequence in the range from position 8309 to position 8341 in the nucleotide sequence of SEQ ID NO:2,
and particularly preferably,
(r11-13-3) a nucleotide sequence having consecutive 10 or more nucleotides, more preferably 17 or more nucleotides, and more preferably 20 or more nucleotides contained in the nucleotide sequence in the range from position 4704 to position 4735 in the nucleotide sequence of SEQ ID NO:2,
(r11-19-3) a nucleotide sequence having consecutive 10 or more nucleotides, more preferably 17 or more nucleotides, and more preferably 20 or more nucleotides contained in the nucleotide sequence in the range from position 2682 to position 2706 in the nucleotide sequence of SEQ ID NO:2,
(r11-21-3) a nucleotide sequence having consecutive 10 or more nucleotides, more preferably 17 or more nucleotides, and more preferably 20 or more nucleotides contained in the nucleotide sequence in the range from position 3549 to position 3578 in the nucleotide sequence of SEQ ID NO:2,
(r11-23-3) a nucleotide sequence having consecutive 10 or more nucleotides, more preferably 17 or more nucleotides, and more preferably 20 or more nucleotides contained in the nucleotide sequence in the range from position 5071 to position 5098 in the nucleotide sequence of SEQ ID NO:2,
(r11-25-3) a nucleotide sequence having consecutive 10 or more nucleotides, more preferably 17 or more nucleotides, and more preferably 20 or more nucleotides contained in the nucleotide sequence in the range from position 6576 to position 6600 in the nucleotide sequence of SEQ ID NO:2,
(r11-27-3) a nucleotide sequence having consecutive 10 or more nucleotides, more preferably 17 or more nucleotides, and more preferably 20 or more nucleotides contained in the nucleotide sequence in the range from position 7941 to position 7971 in the nucleotide sequence of SEQ ID NO:2, or
(r11-29-3) a nucleotide sequence having consecutive 10 or more nucleotides, more preferably 17 or more nucleotides, and more preferably 20 or more nucleotides contained in the nucleotide sequence in the range from position 8312 to position 8341 in the nucleotide sequence of SEQ ID NO:2.

In the nucleotide sequences (r11-13-1) to (r11-29-1), the "nucleotide sequence having consecutive 10 or more nucleotides, more preferably 17 or more nucleotides, and more preferably 20 or more nucleotides" is preferably a "nucleotide sequence having consecutive 10 or more nucleotides, more preferably 17 or more nucleotides, and more preferably 20 or more nucleotides starting from any of the 1 to 12th nucleotide counted from the 5' end, toward the 3' end.

In the sequences (r11-13-2) to (r11-29-2) mentioned above, the "nucleotide sequence having consecutive 10 or more nucleotides, more preferably 17 or more nucleotides, and more preferably 20 or more nucleotides" is preferably a "nucleotide sequence having consecutive 10 or more nucleotides, more preferably 17 or more nucleotides, and more preferably 20 or more nucleotides starting from any of the 1 to 5th nucleotide counted from the 5' end, toward the 3' end.

In the sequences (r11-13-3) to (r11-29-3) mentioned above, the "nucleotide sequence having consecutive 10 or more nucleotides, more preferably 17 or more nucleotides, and more preferably 20 or more nucleotides" is preferably a "nucleotide sequence having consecutive 10 or more nucleotides, more preferably 17 or more nucleotides, and more preferably 20 or more nucleotides starting from any of the 1 to 2nd nucleotide counted from the 5' end, toward the 3' end. The "nucleotide at any of 1 or 2nd nucleotide counted from the 5' end" is more preferably "the nucleotide at the 5' end".

The partial nucleotide sequence f11 and the partial nucleotide sequence r11 are any combination as long as the nucleotide at the 3' end of the partial nucleotide sequence f11 is positioned closer (upstream) to the 5' end of the nucleotide sequence of SEQ ID NO:2 than the nucleotide at the 5' end of the partial nucleotide sequence r11.

Preferably, when the partial nucleotide sequence f11 is (f1 1-12-1), (f11-12-2) or (f11-12-3), the partial nucleotide sequence r11 is (r11-13-1), (r11-13-2), (r11-13-3), (r11-23-1), (r11-23-2) or (r11-23-3). In this case, a partial nucleotide sequence of SEQ ID NO:7 can be amplified.

Preferably, when the partial nucleotide sequence f11 is (f1 1-18-1), (f1 1-18-2) or (f11-18-3), the partial nucleotide sequence r11 is (r11-19-1), (r11-19-2), (r11-19-3), (r11-21-1), (r11-21-2) or (r11-21-3). In this case, a partial nucleotide sequence of SEQ ID NO:5 can be amplified.

Preferably, when the partial nucleotide sequence f11 is (f11-20-1), (f1 1-20-2) or (f11-20-3), the partial nucleotide sequence r11 is (r11-19-1), (r11-19-2), (r11-19-3), (r11-21-1), (r11-21-2) or (r11-21-3). In this case, a partial nucleotide sequence of SEQ ID NO:5 can be amplified.

Preferably, when the partial nucleotide sequence f11 is (f11-22-1), (f1 1-22-2) or (f11-22-3), the partial nucleotide sequence r11 is (r11-13-1), (r11-13-2), (r11-13-3), (r11-23-1), (r11-23-2) or (r11-23-3). In this case, a partial nucleotide sequence of SEQ ID NO:7 can be amplified.

Preferably, when the partial nucleotide sequence f11 is (f11-24-1), (f11-24-2) or (f11-24-3), the partial nucleotide sequence r11 is (r11-25-1), (r11-25-2) or (r11-25-3). In this case, a partial nucleotide sequence of SEQ ID NO:9 can be amplified.

Preferably, when the partial nucleotide sequence f11 is (f11-26-1), (f1 1-26-2) or (f11-26-3), the partial nucleotide sequence r11 is (r11-27-1), (r11-27-2), (r11-27-3), (r11-29-1), (r11-29-2) or (r1 1-29-3). In this case, a partial nucleotide sequence of SEQ ID NO: 11 can be amplified.

Preferably, when the partial nucleotide sequence f11 is (f1 1-28-1), (f1 1-28-2) or (f11-28-3), the partial nucleotide sequence r11 is (r11-27-1), (r11-27-2), (r11-27-3), (r11-29-1), (r11-29-2) or (r11-29-3). In this case, the partial nucleotide sequence of SEQ ID NO: 11 can be amplified.

The polynucleotide of the first primer can be designed such that it contains, at the 3' end, a nucleotide sequence f12 capable of hybridizing to the complementary nucleotide sequence to the partial nucleotide sequence f11. The polynucleotide of the first primer is satisfactory as long as it contains the nucleotide sequence f12 at the 3' end and another nucleotide sequence may further be added to the 5' end side of the nucleotide sequence f12. The full length of the polynucleotide of the first primer is not particularly limited but it can be set at, for example, 10 or more nucleotides, more preferably 17 or more nucleotides and more preferably 20 or more nucleotides, and typically 40 or less nucleotides, 30 or less nucleotides, or 27 or less nucleotides.

When the nucleotide sequence f12 is capable of hybridizing to the complementary nucleotide sequence to the partial nucleotide sequence f11, the partial nucleotide sequence f1 1 and the nucleotide sequence f12 are identical or homologous. The "homologous" herein means that condition (B) or (C) is satisfied, more specifically the following condition (B2) or (C2) is satisfied:
(B2) the nucleotide sequence f12 is a nucleotide sequence in which one or several nucleotides are deleted, substituted, added and/or inserted in the partial nucleotide sequence f11.
(C2) the nucleotide sequence f12 is a nucleotide sequence having an identity of 80% or more to the partial nucleotide sequence f11.

The preferable range of "one or several" in the condition (B2) and the preferable range of the "identity" in the condition (C2) are as described in connection with the condition (B) and (C), respectively.

The polynucleotide of the second primer can be designed such that it contains, at the 3' end, the nucleotide sequence r12 capable of hybridizing to the partial nucleotide sequence r11. The polynucleotide of the second primer is satisfactory as long as it contains the nucleotide sequence r12 at the 3' end and another nucleotide sequence may further be added to 5' end side of the nucleotide sequence r12. The full length of the polynucleotide of the second primer is not particularly limited but it may be, for example, 10 or more nucleotides, more preferably 17 or more nucleotides, more preferably 20 or more nucleotides, and typically 40 or less nucleotides, 30 or less nucleotides, or 27 or less nucleotides.

When the nucleotide sequence r12 is capable of hybridizing to the partial nucleotide sequence r11, the complementary nucleotide sequence to the partial nucleotide sequence r11 and the nucleotide sequence r12 are the same or homologous. The "homologous" herein means that condition (B) or (C) is satisfied, more specifically, the following condition (B3) or (C3) is satisfied:
(B3) the nucleotide sequence r12 is a nucleotide sequence in which one or several nucleotides are deleted, substituted, added and/or inserted in the complementary nucleotide sequence to the partial nucleotide sequence r11.
(C3) the nucleotide sequence r12 is a nucleotide sequence having an identity of 80% or more to the complementary nucleotide sequence to the partial nucleotide sequence r11.

The preferable range of "one or several" in the condition (B3) and the preferable range of the "identity" in the condition (C3) are as defined in connection with the condition (B) and (C), respectively.

A combination of the first primer and the second primer is not particularly limited. The combination can be set such that a target region of a genomic DNA can be amplified by a nucleic acid amplification reaction to obtain a polynucleotide fragment.

A preferable example of a primer set according to the third embodiment is a primer set having
the first primer containing
   (C28f) a polynucleotide having 40 or less nucleotides and containing nucleotide sequence C28fb having consecutive 10 or more nucleotides contained in nucleotide sequence C28fa, which is identical or homologous to the nucleotide sequence of SEQ ID NO: 12, 18, 20, 22, 24, 26 or 28, at the 3' end, and
the second primer containing
   (C28r) a polynucleotide having 40 or less nucleotides and containing nucleotide sequence C28rb having consecutive 10 or more nucleotides contained in nucleotide sequence C28ra, which is identical or homologous to the nucleotide sequence of SEQ ID NO: 13, 19, 21, 23, 25, 27 or 29, at the 3' end.

In the (C28f), nucleotide sequence C28fa is identical or homologous to the nucleotide sequence of SEQ ID NO: 12, 18, 20, 22, 24, 26 or 28, that is, satisfies the condition (B) or (C) (the nucleotide sequence of SEQ ID NO: 12, 18, 20, 22, 24, 26 or 28 corresponds to the nucleotide sequence X and nucleotide sequence C28fa corresponds to nucleotide sequence Y).

In the (C28f), nucleotide sequence C28fa is preferably a nucleotide sequence satisfying that
part of a sequence consisting of preferably consecutive 3 or more nucleotides, more preferably 5 or more nucleotides, more preferably 10 or more nucleotides, more preferably 12 or more nucleotides, more preferably 15 or more nucleotides, more preferably 17 or more nucleotides, and more preferably 20 or more nucleotides from the 3' end is identical to the nucleotide sequence of SEQ ID NO: 12, 18, 20, 22, 24, 26 or 28, the rest is homologous (namely, the condition (B) or (C) is satisfied (the rest of the nucleotide sequence SEQ ID NO: 12, 18, 20, 22, 24, 26 or 28 corresponds to nucleotide sequence X, and the rest of nucleotide sequence C28fa corresponds to nucleotide sequence Y)); and preferably identical to the nucleotide sequence of SEQ ID NO: 12, 18, 20, 22, 24, 26 or 28.

Nucleotide sequence C28fb is preferably a nucleotide sequence having consecutive 12 or more nucleotides, 15 or more nucleotides, 17 or more nucleotides, or 20 or more nucleotides, contained in nucleotide sequence C28fa, and more preferably, a nucleotide sequence continued from the 3' end of nucleotide sequence C28fa.

Nucleotide sequence C28fb is more preferably identical to nucleotide sequence C28fa.

In the (C28r), nucleotide sequence C28ra is identical or homologous to the nucleotide sequence of SEQ ID NO: 13, 19, 21, 23, 25, 27 or 29, that is, satisfies the condition (B) or (C) (the nucleotide sequence of SEQ ID NO: 13, 19, 21, 23, 25, 27 or 29 corresponds to nucleotide sequence X, whereas nucleotide sequence C28ra corresponds to nucleotide sequence Y).

In the (C28r), nucleotide sequence C28ra, preferably a nucleotide sequence satisfying that part of a sequence consisting of preferably 3 or more nucleotides, more preferably 5 or more nucleotides, more preferably 10 or more nucleotides, more preferably 12 or more nucleotides, more preferably 15 or more nucleotides, more preferably 17 or more nucleotides, and more preferably 20 or more nucleotides from the 3' end is identical to the nucleotide sequence of SEQ ID NO: 13, 19, 21, 23, 25, 27 or 29; and the rest is homologous (namely, the condition (B) or (C) is satisfied (the rest of the nucleotide sequence SEQ ID NO: 13, 19, 21, 23, 25, 27 or 29 corresponds to nucleotide sequence X, the rest of nucleotide sequence C28ra corresponds to nucleotide sequence Y)), and preferably identical to the nucleotide sequence of SEQ ID NO: 13, 19, 21, 23, 25, 27 or 29.

Nucleotide sequence C28rb is preferably a nucleotide sequence having consecutive 12 or more nucleotides, 15 or more nucleotides, 17 or more nucleotides, or 20 or more nucleotides contained in nucleotide sequence C28ra, and more preferably, a consecutive nucleotide sequence from the 3' end of nucleotide sequence C28ra.

Nucleotide sequence C28rb is more preferably identical to nucleotide sequence C28ra.

The first primer may consist of only the polynucleotide described above, or may comprise, in addition to the polynucleotide, a label portion or a binding portion described below. The polynucleotide and the label portion or binding portion can be chemically linked via an appropriate spacer described below. In the first primer, the position at which one of the label portion and the binding portion is linked to the polynucleotide is not particularly limited as long as the position does not inhibit annealing of the polynucleotide and a polynucleotide comprising a target region or a complementary strand thereof and elongation in a nucleic acid amplification reaction, but it is preferably the 5' end of the polynucleotide.

The second primer may consist of only the polynucleotide described above, or may comprise, in addition to the polynucleotide, a label portion or a binding portion described below. The polynucleotide and the label portion or binding portion can be chemically linked via an appropriate spacer described below. In the second primer, the position at which one of the label portion and the binding portion is linked to the polynucleotide is not particularly limited as long as the position does not inhibit annealing of the polynucleotide and a polynucleotide comprising a target region or a complementary strand thereof and elongation in a nucleic acid amplification reaction, but it is preferably the 5' end of the polynucleotide.

When at least one of the first primer and the second primer comprises a label portion, detection of amplified products is easy because a nucleic acid amplification reaction using such a primer yields amplified products comprising the label portion.

When at least one of the first primer and the second primer comprises a binding portion, an amplified product by a nucleic acid amplification reaction using the primer set contains a binding portion, and therefore, the amplified product can be fixed to a solid phase support and easily detected.

More preferably, one of the first primer and the second primer further comprises a label portion and the other one further comprises a binding portion. In a nucleic acid amplification reaction using the primer set according to the embodiment, a double stranded amplified product containing the label portion and the binding portion can be obtained, and therefore, detection by nucleic acid chromatography can be easily made.

Hereinafter, a set of primers comprising a label portion and a binding portion will be described.

### (Label Portion)

The label portion is either a label substance or a tag capable of binding to a label substance, and preferably a tag capable of binding to a label substance. In this description, the tag capable of binding to a label substance is sometimes referred to as a labelling tag. When the label portion is a labelling tag, an amplified product obtained by a nucleic acid amplification reaction can be contacted with a label substance to label the tag contained in an end of the amplified product. When the label portion is a label substance, an amplified product containing a label substance in an end can be obtained as the amplified product of the nucleic acid amplification reaction.

The label substance is not particularly limited as long as it makes the amplified product detectable, but it is preferably a substance that makes the amplified product visually detectable. Examples of such a label substance include a colored particle, a dye, an enzyme (peroxidase, alkaline phosphatase, luciferase, and the like) and it is preferably a colored particle. Examples of the "colored particle" include, but are not limited to, a metal (for example, gold, silver, copper, and platinum) particle, a metal rod, a colored latex particle, and a dye-containing silica nanoparticle. The size of the label substance is not limited as long as the size does not prevent trapping of the amplified product to a solid phase support described below. It is preferred that the label substances have good color development upon detection. The size of the label substance can be selected appropriately so as to be smaller than the pore size of a solid phase support described below or various porous member of the nucleic acid detection device comprising a solid support. For example, the size of label substance may be about 500 nm or less, preferably about 0.1 nm to 250 nm, more preferably about 1 nm to 100 nm in particle diameter. As the dye, fluorescent dyes (cyanine, fluorescein, and the like) can be used, and in that case, it is preferred to irradiate with excitation wavelength light of each fluorescent dye to detect.

The labelling tag that can be used as the label portion is not particularly limited as long as it is capable of binding to a label substance, and can be selected appropriately depending on the structure of the label substance. Examples of such a tag include a nucleic acid (a DNA, an RNA, or the like), a protein, a peptide or other compound (e.g., biotin, fluorescein isothiocyanate (FITC), low molecular weight compound such as digoxigenin (DIG)), and a combination thereof. In one preferred embodiment, the labelling tag comprises or consists of a polynucleotide. The polynucleotide that can be comprised in a labelling tag is not particularly limited as long as it does not substantially inhibit the nucleic acid amplification reaction by the primer set. A polynucleotide having, e.g., 5 to 50, preferably 10 to 35 nucleotides, and more preferably, a polynucleotide comprising (or consisting of) a nucleotide sequence of SEQ ID NO:14 or 15 or a partial nucleotide sequence or a complementary nucleotide sequence thereof, can be used. Another preferred embodiment of the labelling tag is a tag made of a small molecule compound such as biotin, FITC or DIG.

When a label portion is the labelling tag described above, a label substance may be directly or indirectly bound to the labelling tag, and a suitable binding means can be selected appropriately depending on the combination of the label substance and the labelling tag to be used. For example, when the labelling tag comprises a polynucleotide, a label substance may be indirectly bound to the labelling tag by binding the label substance to a polynucleotide capable of hybridizing to the polynucleotide comprised in the labelling tag (for example, a polynucleotide comprising a sequence complementary to the nucleotide sequence of the polynucleotide comprised in the labelling tag), and causing hybridization between the two polynucleotides. Binding of the label substance to the polynucleotide may be performed via a peptide, a protein, a nucleic acid or the like, and may be performed via a suitable functional group. Conditions of hybridization are not particularly limited as long as it causes hybridization, but, for example, a hybridization can be performed by allowing a reaction at 20 to 40°C in a buffer containing 10 to 50 mM phosphate (pH 6 to 7). To enhance the efficiency of hybridization, the buffer may further contain a salt such as sodium chloride.

When the labelling tag is a low molecular weight compound, labelling can be performed with a label substance coupled to a binding agent such as a protein (such as avidin that binds to biotin, a protein that binds to FITC), an antibody (e.g., anti-DIG antibody), and an aptamer which specifically binds to the compound. In this case, the labelling tag can be bound to the binding substance using various buffers near neutral.

The label portion (labelling tag or label substance) can be bound to the polynucleotides contained in the first primer or the polynucleotides contained in the second primer, via any means, and directly or indirectly. However, when at least a part in the label portion connecting it to the polynucleotide is composed of a polynucleotide, the polynucleotide and the label portion are bound via a spacer capable of suppressing or halting a progression of a DNA polymerase reaction so as not to form a double strand of the part and the polynucleotide due to a nucleic acid amplification reaction. Such a "spacer" is not limited as long as it can suppress or halt the progression of a DNA polymerase reaction and prevents the label portion from being double stranded. Examples of such a "spacer" include, but not limited to, a nucleic acid sequence having a strong hairpin structure and/or a pseudoknot structure, an L-type nucleic acid, a peptide nucleic acid (PNA), a cross-linked nucleic acid (bridged nucleic acid (BNA) or locked nucleic acid (LNA)), fluorescein, Cy3, Cy5, a divalent group containing an azobenzene structure represented by the following formula I, aliphatic chain (alkylene chain or polyoxyalkylene chain), a divalent group containing inverted sequence structure such as 5'-5' and 3'-3' linkages.

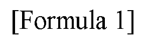

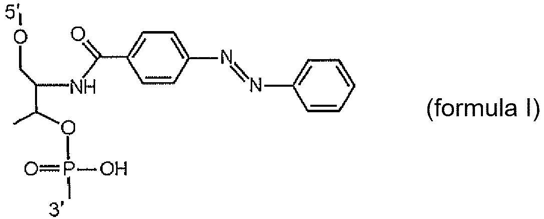

When two polynucleotide molecules are connected via a divalent group represented by formula I, the phosphate group of one end of the divalent group, that is, the 3' end of the divalent group, refers to a phosphate group of the 5' end nucleotide of one of the polynucleotide molecules, and an oxygen atom of the other end, that is, the 5' end, forms a phosphoric acid ester bond together with the phosphate group at the 3'-end nucleotide of the other polynucleotide molecule.

The spacer of an aliphatic chain, includes, for example, a spacer represented by the following formula (II):

5'-O-CₘH₂ₘ-O-3' formula (II)

wherein 5' represents an oxygen atom of a phosphodiester bond on the 5' side and 3' represents an oxygen atom of a phosphodiester bond on the 3' side and m represents an integer of 1 or more and 40 or less, and H may be substituted by a substituent.

In formula (II), m is preferably 2 or more and 36 or less, more preferably 3 or more and 16 or less. H in formula (II) may be substituted by a substituent, and examples of the substituent typically include an alkyl group, an alkoxy group, and a hydroxyl group. The number of carbon atoms in the alkyl group and alkoxy group as the substituent is preferably 1 to 8, more preferably 1 to 4. When there are 2 or more substituents, the substituents may be the same or different. The spacer of formula (II) with no substituents is also preferred.

The other spacers include a spacer represented by the following formula (III):

5'-(OCₙH₂ₙ)_{L}-O-3' formula (III)

wherein 5' represents an oxygen atom of a phosphodiester bond on the 5' side and 3' represents an oxygen atom of a phosphodiester bond on the 3' side, n represents an integer of 2 or more and 4 or less, L represents an integer of 1 or more, and (n+1) × L represents an integer of 40 or less, and H may be substituted by a substituent.

In formula (III), (n + 1) × L is preferably 2 or more and 36 or less, more preferably 3 or more and 16 or less. The same embodiments apply to the substituents of H in formula (III) as the substituents in formula (II).

Other examples of aliphatic chain spacers include the following divalent groups.

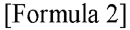

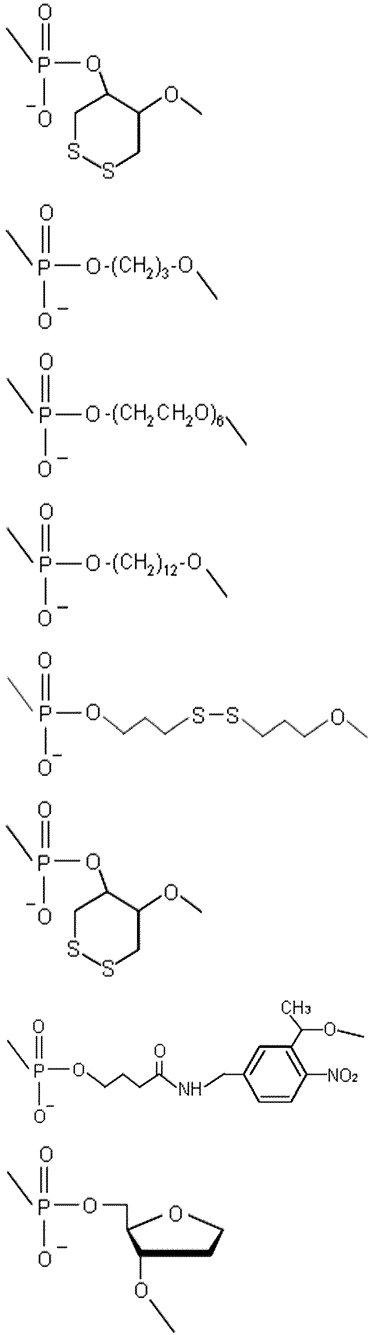

When connecting two polynucleotide molecules via these divalent groups, the phosphate group of one end of each divalent group refers to a phosphate group of a nucleotide at the 3' end or 5' end of one polynucleotide molecule, and an oxygen atom of the other end, together with the phosphate group of a nucleotide at the 5' end or 3' end of the other polynucleotide molecule, forms a phosphoric acid ester bond.

### (Binding Portion and Solid Phase Support)

The binding portion is a tag capable of binding to a solid phase support described below. In this description, the tag capable of binding to a solid phase support is sometimes referred to as a fixing tag.

The fixing tag which can be used as the binding portion is not particularly limited as long as it is capable of binding to a solid phase support, and can be selected appropriately depending on the structure of the solid phase support. Examples of such a fixing tag include a polynucleotide (a DNA, an RNA, or the like), a protein, a peptide or other compound (e.g., a low molecular weight compound) and a combination thereof. In one preferred embodiment, the fixing tag comprises or consists of a polynucleotide. The polynucleotide that can be contained in a fixing tag is not particularly limited as long as it does not substantially inhibit a nucleic acid amplification reaction by the primer set. A polynucleotide having, e.g., 5 to 50, preferably 10 to 35 nucleotides, and more preferably, a polynucleotide comprising(or consisting of) a nucleotide sequence of SEQ ID NO: 14 or 15 or a partial nucleotide sequence or a complementary nucleotide sequence thereof can be used.

The solid phase support is not particularly limited, and it may be made of resin, metal, polysaccharide, mineral or the like, and it may be in the form of a membrane, a film, a nonwoven fabric, a plate, a gel, and the like. Preferably, the solid phase support is one having a porous structure so as to allow development of the amplified product and/or the label substance in the solution. Examples of the solid phase support that can be used in the present invention include a filter paper, a nitrocellulose membrane, a polyethersulfone membrane, a nylon membrane, dried various gel (silica gel, agarose gel, dextran gel, gelatin gel), silicon, glass, and plastic. The size and shape of the solid phase support can be selected appropriately so as to be suitable for various operations and detection.

The solid phase support is sufficient if it is configured to allow binding of at least a part of the solid phase support to the fixing tag, and it is more preferred that it is configured to allow binding of only a part of the solid phase support to the fixing tag. By configuration to allow binding of only a certain part of the solid phase support to the fixing tag, it becomes easier to determine positive or negative since the amplified product captured by the solid phase support is detected only at the certain part.

The solid phase support may bind directly or indirectly to the fixing tag, and a suitable binding means can be selected appropriately depending on the combination of the solid phase support and the fixing tag to be used. For example, when the fixing tag comprises a polynucleotide, a solid phase support may be indirectly bound to the fixing tag by fixing a polynucleotide capable of hybridizing to the polynucleotide comprised in the solid phase support (for example, a polynucleotide comprising a sequence complementary to the nucleotide sequence of the polynucleotide comprised in the solid phase support) on the solid phase support to provide a tag capture means, and causing hybridization between the two polynucleotides. Fixing of the polynucleotide on the solid phase support may be carried out via a peptide, a protein, a nucleic acid or the like, and may be carried out via a suitable functional group. Hybridization can be performed under the condition as described above with respect to the binding of the labelling tag to the label substance. When fixing a polynucleotide on a solid phase support, by fixing it only to a certain part, it becomes easier to determine positive or negative since the captured amplified product is detected only at the certain part.

The binding portion (fixing tag) can bind to a polynucleotide contained in the first primer or a polynucleotide contained in the second primer by any means, and directly or indirectly. However, when at least a part in the fixing tag connecting it to the polynucleotide is composed of a polynucleotide, the polynucleotide and the fixing tag are bound via a spacer capable of suppressing or halting a progression of a DNA polymerase reaction so that the part will not be double stranded together with the polynucleotide by a nucleic acid amplification reaction. Specific examples of the spacer provided between the binding portion and the polynucleotide are the same as those described above with respect to the spacers provided between the label portion and the polynucleotide.

### <Method for Determining Genotype Using Primer Set for Detecting Indicator Nucleotide Sequence Specific to C28 Strain>

It is possible to determine whether the base corresponding to position 28 in the nucleotide sequence of SEQ ID NO:1 of erm (41) gene is cytosine or thymine in a mycobacterium belonging to *Mycobacteroides abscessus* complex by using a primer set according to the third embodiment containing the first primer and the second primer.

The determination method comprises, for example,
performing a nucleic acid amplification reaction using a genomic DNA of a mycobacterium as a subject to be determined or a polynucleotide derived from the genomic DNA of a mycobacterium as a subject to be determined as a template and a primer set according to the third embodiment containing the first primer and the second primer,
detecting an amplified product obtained by the nucleic acid amplification reaction, and
determining that the base in erm (41) gene in the genomic DNA of the mycobacterium is cytosine when the amplified product is detected, and that the base is thymine when the amplified product is not detected.

A primer set according to the third embodiment containing the first primer and the second primer can be used in a method for determining whether the base corresponding to position 28 in the nucleotide sequence of SEQ ID NO:1 of erm (41) gene is cytosine in a mycobacterium. This method comprises, for example,
performing a nucleic acid amplification reaction using a genomic DNA of a mycobacterium as a subject to be determined or a polynucleotide derived from the genomic DNA of a mycobacterium as a subject to be determined as a template and a primer set according to the third embodiment containing the first primer and the second primer,
detecting an amplified product obtained by the nucleic acid amplification reaction, and
determining that the base in erm (41) gene in the genomic DNA of the mycobacterium is cytosine when the amplified product is detected.

Examples of the "polynucleotide derived from a genomic DNA of a mycobacterium as a subject to be determined" herein include a partial polynucleotide, which is part of the genomic DNA and an amplified product obtained by amplifying the whole genomic DNA or a part thereof containing the indicator nucleotide sequence by a nucleic acid amplification reaction. Examples of the nucleic acid amplification reaction are as described above.

When a genomic DNA of a mycobacterium as a subject to be determined or a polynucleotide derived from the genomic DNA of a mycobacterium as a subject to be determined contains a target sequence of a nucleic acid amplification reaction using the first primer and the second primer, a polynucleotide fragment containing the target sequence is produced as an amplified product.

A method for detecting an amplified product produced by a nucleic acid amplification reaction is not particularly limited. Examples thereof include a method in which a reaction solution of a nucleic acid amplification reaction is subjected to fractionation by gel electrophoresis and the presence or absence of a band having a size equivalent to that of a polynucleotide fragment containing the predetermined target sequence is confirmed; and a method in which a labeled polynucleotide probe specific to a polynucleotide fragment containing a predetermined target sequence is hybridized to a reaction solution of a nucleic acid amplification reaction or a product thereof to detect a complex.

A nucleic acid amplification reaction is caried out in the presence of a probe according to the second embodiment of the present invention having a reporter fluorescent substance linked at the 5' end thereof (of the polynucleotide of the probe) and a quencher dye linked at the 3' end, and a polynucleotide fragment is detected using fluorescence, which is generated when a polynucleotide fragment containing a predetermined target sequence is amplified, as an indicator. This is also an embodiment of a method for detecting an amplified product.

When one of the first primer and the second primer further comprises a label portion that is a tag capable of binding to a label substance or a label substance, and the other further comprises a binding portion that is a tag capable of binding to a solid phase support, the following detection step using the solid phase support can be employed.

### (Detection step using solid phase support)

In the detection step, a product of a nucleic acid amplification reaction is brought into contact with a solid phase support containing a portion capable of binding to a binding portion at least in part, and an amplified product is detected at the aforementioned portion of the solid phase support using the labelling portion as an indicator.

When the label portion is a labelling tag described above, a labelling step of binding the label substance to the labelling tag may be further performed. Details of the labelling step are described below. When the label portion is a label substance described above, the labelling step is not necessary. The phrase "using a label portion as an indicator" in the detection step refers to, when the label portion is a labelling tag, detecting the amplified product using a label substance bound through the labelling step as an indicator, and when the label portion is a label substance, detecting the amplified product using the label substance as an indicator.

The product of a nucleic acid amplification reaction refers to a reaction solution of a nucleic acid amplification reaction possibly containing an amplified product, a sample obtained by further concentrating the amplified product from the reaction solution, or the like.

Details of the solid phase support are as described above.

Contact of the portion capable of binding to a binding portion in a solid phase support with the product can be performed depending on the combination of the solid phase support and the binding portion, and it can be performed in a condition appropriately adjusted so as to allow binding between the binding portion of the amplified product and the portion capable of binding to the binding portion (e.g. hybridization conditions or a buffer condition of about pH 5 to 9) when the amplified product is contained in the product,.

Detection of the amplified products can be carried out by detecting, preferably visually detecting, the label substance bound to the amplified product captured on a solid phase support. When the amplified product is present, the label substance of the amplified product captured and bound by the solid phase support is detected. It is possible to determine the presence or absence of an amplified product (polynucleotide fragment containing the target sequence) in the reaction system of a nucleic acid amplification reaction using the presence or absence of the detection as an indicator.

### (Labelling step)

The labelling step is a step which is carried out when the label portion contained in the primer set is a labelling tag as described above. The step is carried out by bringing a product of the nucleic acid amplification reaction into contact with the label substance to allow the labelling substance to bind to the labelling tag. The labelling step may be performed before, after, or at the same time that a product of the nucleic acid amplification reaction is brought into contact with a solid phase support.

Contact of the label substance with the product can be performed in a condition appropriately adjusted so as to allow binding between the label substance and the labelling tag of the amplified product (e.g. aforementioned hybridization conditions or a buffer condition of about pH 5 to 9) when the amplified product is contained in the product, depending on the combination of the label substance and the labelling tag.

### (Detection device)

The detection step and labelling step described above can be carried out by a nucleic acid detection device using nucleic acid chromatography. By using the nucleic acid detection device, it is possible to detect or determine the presence or absence of an amplified product (polynucleotide fragment containing a target sequence) in the reaction system of a nucleic acid amplification reaction, without requiring special equipment, and the results can be easily and quickly obtained.

The nucleic acid detection device may be a known nucleic acid detection device used to detect the labeled nucleic acid amplified product by a nucleic chromatography method (WO2012/070618).

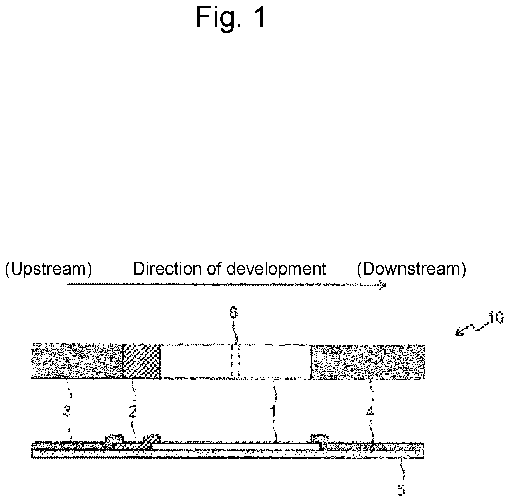

The schematic diagram of one embodiment of the nucleic acid detection device available in the present invention is shown in Figure 1, but a nucleic acid detection device is not limited to this embodiment. In the following description, reference signs for each member correspond to reference signs shown in Figure 1.

The nucleic acid detection device 10 in Figure 1 is formed by disposing, on a substrate 5, a sample pad 3 which is a reaction system receiving portion for receiving a reaction system of the nucleic acid amplification reaction, a conjugate pad 2 that holds a label substance, a porous solid phase support 1 comprising in a part a portion 6 capable of binding to a binding portion contained in the amplified product and an absorbent pad 4 so that they contact with each other in this order. The portion 6 of the solid phase support 1 is a portion where the means for capturing the amplified product (capture means) (e.g., an oligonucleotide as mentioned above) is locally disposed and fixed. The surface of the solid phase support 1 may be covered with a film (not shown in the figure). The sample pad 3, the conjugate pad 2, the solid phase support 1 and the absorbent pad 4 can be constituted of a member having a porous structure available as the solid phase support described above. These may be constituted of the same member or different members. The substrate 5 is only required that it can support various members disposed thereon and facilitates manipulation of the nucleic acid detection device. Examples of such a substrate include one made of a resin, a metal, a mineral, or the like. The conjugate pad 2 can be omitted when the label substance is mixed into a development solution or when the label portion is a label substance.

A reaction system of the nucleic acid amplification reaction is added to the sample pad 3. The reaction system may be added as it is, or may be added together with a suitable development solution (e.g., phosphate buffer, Tris buffer, Good's buffer, SSC buffer). If necessary, the development solution may further comprise a surfactant, a salt, a protein, a nucleic acid or the like. The reaction system added to the sample pad 3 is developed by a capillary phenomenon from the upstream toward the downstream in the direction indicated by the arrow in Figure 1.

As another embodiment, it can be developed by putting the nucleic acid detection device in the container holding the product of the nucleic acid amplification reaction and/or the development solution (e.g., PCR tube, Eppendorf tube, 96 well plate), and immersing the sample pad 3 in the product of the nucleic acid amplification reaction and/or the development solution. In that case, the width of the sample pad 3 is preferably set to 2.0 to 10.0 mm, more preferably 2.0 to 5.0 mm so that the sample pad 3 can be put in the container holding the product of the nucleic acid amplification reaction and/or the development solution.

In the embodiment where the label portion is a labelling tag, the amplified product in the reaction system contacts with a label substance when passing through the conjugate pad 2 holding a label substance, and is labeled with a label substance via a labelling tag.

Then, the amplified product in the reaction system contacts with a capture means fixed to the portion 6 when passing through the solid phase support 1, and is captured and bound to the solid phase support 1 via a fixing tag.

When the amplified product is present, the label substance bound to the amplified product, which is captured/bound at the portion 6 of the solid phase support 1 having a capturing means is detected at the portion 6. If the label substance can be visually observed, the portion 6 is colored due to the label substance. The presence or absence of an amplified product in a sample can be determined using the presence or absence of detection (coloration) of the label substance as an indicator.

### <Method for determining sensitivity to macrolide antibiotic substance using primer set for detecting indicator nucleotide sequence specific to C28 strain>

Using the primer set according to the third embodiment containing the first primer and the second primer, it is possible to determine sensitivity of a mycobacterium, in particular, a mycobacterium belonging to *Mycobacteroides abscessus* complex, to a macrolide antibiotic substance.

The detection method comprises for example,
performing a nucleic acid amplification reaction using a genomic DNA of a mycobacterium as a subject to be determined or a polynucleotide derived from the genomic DNA of a mycobacterium as a subject to be determined as a template, and a primer set according to the third embodiment containing the first primer and the second primer,
detecting an amplified product obtained by the nucleic acid amplification reaction, and
determining that the mycobacterium has a sensitivity to a macrolide antibiotic substance when the amplified product is detected, and that the mycobacterium does not have a sensitivity to a macrolide antibiotic substance when the amplified product is not detected.

In the determination method, the nucleic acid amplification reaction and detection of an amplified product can be carried out in the same manner as mentioned above.

### <Method 2 for Determining Genotype of erm (41) Gene of Mycobacterium>

A fourth embodiment of the present invention relates to a method for determining whether the base corresponding to position 28 in the nucleotide sequence of SEQ ID NO:1 of erm (41) gene is cytosine or thymine in a mycobacterium belonging to *Mycobacteroides abscessus* complex, comprising
detecting, in a genomic DNA of the mycobacterium as a subject to be determined, an indicator nucleotide sequence, which is present in the region except erm (41) gene in the genomic DNA when the base in erm (41) gene is thymine, and which is not present when the base is cytosine, wherein
detection of the indicator nucleotide sequence indicates that the base in erm (41) gene in the genomic DNA of the mycobacterium is cytosine, and
non-detection of the indicator nucleotide sequence indicates that the base in erm (41) gene in the genomic DNA of the mycobacterium is cytosine.

### Examples

The genomic information of a standard strain of *Mycobacteroides abscessus* complex was obtained from the NCBI database (https://www.ncbi.nlm.nih.gov/). The genomic information of clinically isolated strains and purchased strains of *Mycobacteroides abscessus* complex (shown in the following Table 5) was each obtained by analyzing DNAs prepared from pure cultured bacterial cells by a next-generation sequencer (MiSeq (illumina)). Based on the genomic information, strains (T28 strains) having a normal type (T28) of erm (41) gene and strains (C28 strains) having a mutant type (C28) of erm (41) gene were subjected to molecular phylogenetic analysis. As a result, it was found that T28 strains and C28 strains can be classified into two molecular phylogenetically different groups. Further, alignment of nucleic acid sequences was carried out by use of Mauve software (Darling et al. (2005)) and comparison analysis was carried out. As a result, it was found that C28 strains have the insert nucleotide sequence of SEQ ID NO:2 highly conserved specifically to C28 strains.

The nucleotide sequence of SEQ ID NO:2 will be sometimes referred to as the "insert nucleotide sequence".

### <Example 1>

As a primer set specifically hybridizing to the insert nucleotide sequence of SEQ ID NO:2, C28-f primer and C28-r primer were designed. The sequences of individual primers are shown in Table 2. PCR was performed using the designed primer set, and a nucleic acid amplified product was analyzed by nucleic acid chromatography. In this manner, whether C28 strains can be specifically detected or not was verified.

### (I) Structure of Primer

For detection by nucleic acid chromatography, the DNA consisting of the following tag sequence was linked to the 5' end of each primer via a divalent group represented by formula I containing an azobenzene structure that inhibits a polymerase reaction. At this time, the 5' end of the divalent group containing an azobenzene structure represented by formula I, and the phosphate group of the 3' end nucleotide of the DNA consisting of the tag sequence form a phosphoric acid ester bond; and the phosphate group at the 3' end of the divalent group and the hydroxyl group at position 5' of deoxyribose of the 5' end nucleotide of the primer portion of the primer form a phosphoric acid ester bond.

To the 5' end of C28-f primer consisting of the nucleotide sequence of SEQ ID NO: 12, fixing tag 1 consisting of the nucleotide sequence of SEQ ID NO: 15 was added via the azobenzene structure. To the 5' end of C28-r primer consisting of the nucleotide sequence of SEQ ID NO: 13, labelling tag 1 consisting of the nucleotide sequence of SEQ ID NO: 14 was added, in the same manner.

A combination of C28-f primer and C28-r primer to which the tag was added was used as a first primer set.

The nucleotide sequence of SEQ ID NO:12 is a partial nucleotide sequence of position 4634 to position 4658 in SEQ ID NO:2 and a partial nucleotide sequence of position 581 to position 605 in SEQ ID NO:7.

The nucleotide sequence of SEQ ID NO: 13 is a complementary nucleotide sequence to a partial nucleotide sequence of position 4704 to position 4730 in SEQ ID NO:2 and a complementary nucleotide sequence to a partial nucleotide sequence of position 651 to position 677 in SEQ ID NO:7.

**[Table 2]**

| Name | Sequence | SEQ ID NO |
|---|---|---|
| C28-f | TCCTCGGAATCGGCACTGTCCGTTG | 12 |
| C28-r | TACAGCAGCTCAACAGTGACACCGAAG | 13 |
| Labelling tag 1 | TGGCAACATTTTTCACTGGGTTTATAG | 14 |
| Fixing tag 1 | TGCATCGAGTGACAGCTAATGTGTGAT | 15 |

### (II) Preparation of Oligonucleotide Bound Gold Colloid

Gold Colloid (40 nm, 9.0 × 10¹⁰ (particles/ml), manufactured by British Biocell International, Inc.) were mixed with a thiol group-containing oligonucleotide having the nucleotide sequence of SEQ ID NO: 16 shown in Table 3, and the mixture was incubated at 50°C for 16 hours. After centrifugation for 15 minutes at 6000 rpm, the supernatant was removed, and 0.05 M sodium chloride and 5 mM phosphate buffer (pH 7) were added. After stirring, the obtained mixture was incubated again at 50°C for 40 hours.

After incubation, centrifugation (6000 rpm, 15 minutes) was performed. The supernatant was removed, and 5 mM phosphate buffer (pH 7) was added. This buffer replacement was performed again.

In this manner, an oligonucleotide bound colloidal gold was prepared.

The suspension of the oligonucleotide bound colloidal gold prepared was added to a pad made of fiberglass so as to uniformly spread, and dried in a vacuum dryer to obtain a conjugate pad.

### (Thiol Group-Containing Oligonucleotide)

The thiol group-containing oligonucleotide is an oligonucleotide in which the phosphate group at position 3' at the 3' end of an oligonucleotide consisting of the nucleotide sequence of SEQ ID NO: 16 is linked to the hydroxyl group of a compound represented by HO-(CH₂)ₘ-SH (wherein m is 6) via a phosphoric acid ester bond.

### (III) Preparation of Membrane having Tag Capture Means fixed thereto

A solution containing oligonucleotide probe 1 consisting of the nucleotide sequence of SEQ ID NO: 17 serving as a tag capture means, was applied onto a nitrocellulose membrane (Hi-Flow180) manufactured by Merck Millipore by use of a dispenser in a line of 1 mm in width perpendicular to the direction of development. Thereafter, the membrane was dried at 40°C for 30 minutes to obtain a membrane having a tag capture means. The line to which oligonucleotide probe 1 of SEQ ID NO: 17 was applied, was designated as T1 line.

**[Table 3]**

| Name | Sequence | SEQ ID NO |
|---|---|---|
| Thiol group-containing oligonucleotide | CTATAAACCCAGTGAAAAATGTTGCCA-SH | 16 |
| Oligonucleotide probe 1 | ATCACACATTAGCTGTCACTCGATGCA | 17 |

### (IV) Preparation of Nucleic Acid Detection Device

A nucleic acid detection device was prepared in accordance with the detection device shown in the schematic view of Figure 1.

To describe more specifically, a polypropylene backing sheet (by Lohmann) as the substrate 5, the conjugate pad prepared in above step (II) as the conjugate pad 2, the membrane (solid phase support) 1 having oligonucleotide probe 1 as the tag capture means on the portion 6 prepared in above (III), a sample pad formed of fiberglass as the sample pad 3, and a cellulosic absorbent pad as the absorbent pad 4 were stuck together such that they were overlapped each other as shown in Figure 1 to prepare a nucleic acid detection device 10.

### (V) PCR

Using the first primer set with a tag described in the above section (I), the following PCR reaction solutions were prepared in accordance with the manual of TaKaRa TaqHS perfect Mix.

**[Table 4]**

| | |
|---|---|
| 2x TaKaRa TaqHS perfect Mix | 10.0 µl |
| First primer set | |
| 5 µM Fw primer (SEQ ID NO:12) | 0.5 µl |
| 5 µM Rv primer (SEQ ID NO:13) | 0.5 µl |
| Template DNA (1 ng/µl) | 1.0 µl |
| Sterile distilled water | 8.0 µl |

As template DNAs, DNAs purified from each of the mycobacterial strains (*Mycobacteroides abscessus* complex and clinically isolated strains and purchased strains of other mycobacteria, related mycobacterial strains) shown in Figure 5 were used. As a negative control, a PCR reaction solution having the same composition except that sterilized distilled water (1 µL) was used in place of a solution of a template DNA mentioned above, was prepared. A 5 µM Fw primer solution and a 5 µM Rv primer solution were prepared by dissolving a predetermined primer in sterilized distilled water so as to obtain a concentration of 5 µM.

The PCR reaction solutions were set in a PCR apparatus (LifeEco, by Bioer). After a reaction was carried out at 94°C for one minute, a cycle consisting of a reaction at 94°C for 5 seconds, a reaction at 62°C for 10 seconds and a reaction at 72°C for 5 seconds, was repeated 35 times.

Note that, the *Mycobacteroides abscessus* complex strains used in the assay were subjected to sequencing analysis to identify whether each of them is a T28 strain or a C28 strain.

### (VI) Detection Using Nucleic Acid Chromatographic Detection System

After PCR was carried out in the aforementioned conditions, the reaction solutions were applied to the nucleic acid detection device 10 to detect nucleic acid amplified products. To describe more specifically, 5 µL of each of the reaction solutions after PCR was added to the sample pad 3 on the device 10, and further, 80 µL of a development solution (citrate buffer containing a surfactant) was added to develop the reaction solution. After 10 minutes at room temperature, whether T1 line of the portion 6 containing a tag capture means linearly fixed on the membrane 1 was colored or not, was visually observed.

When coloration of T1 line is observed, it is shown that the insert nucleotide sequence represented by SEQ ID NO:2, which is specifically present in a C28 strain, is contained in a template DNA, and it is demonstrated that a nucleic acid amplified product derived from a C28 strain was obtained.

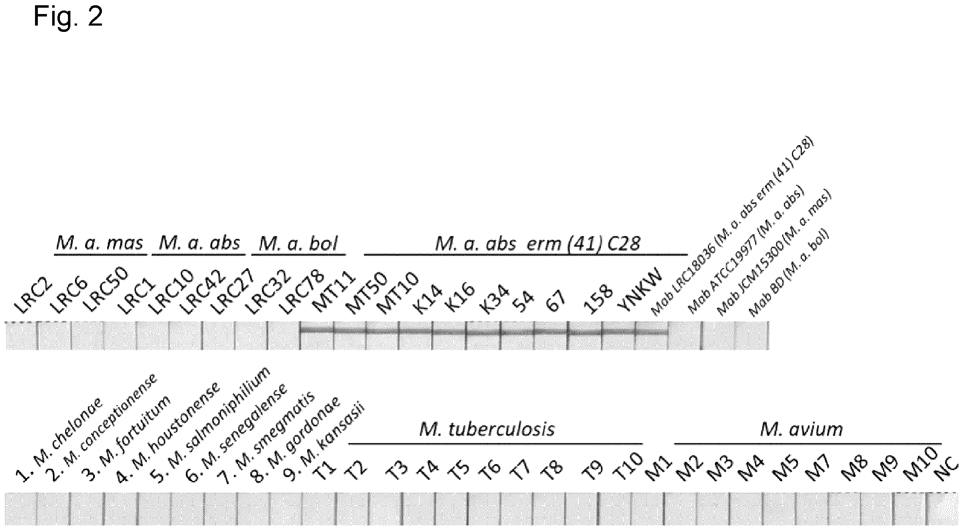

The results are shown in Table 5 (Table 5-1 and Table 5-2) and Figure 2. In Table 5, the cases where coloration was observed was indicated by "+" and the cases where coloration was not observed was indicated by "-".

**[Table 5-1]**

| No. | Name of strain | Analysis results by DNA sequencing | Nucleic acid chromatography Line coloration |
|---|---|---|---|
| 1 | M. abscessus LRC2 | T28 | - |
| 2 | M. abscessus LRC6 | T28 | - |
| 3 | M. abscessus LRC50 | T28 | - |
| 4 | M. abscessus LRC1 | T28 | - |
| 5 | M. abscessus LRC10 | T28 | - |
| 6 | M. abscessus LRC42 | T28 | - |
| 7 | M. abscessus LRC27 | T28 | - |
| 8 | M. abscessus LRC32 | T28 | - |
| 9 | M. abscessus LRC78 | T28 | - |
| 10 | M. abscessus MT11 | C28 | + |
| 11 | M. abscessus MT50 | C28 | + |
| 12 | M. abscessus MT10 | C28 | + |
| 13 | M. abscessus K14 | C28 | + |
| 14 | M. abscessus K16 | C28 | + |
| 15 | M. abscessus K34 | C28 | + |
| 16 | M. abscessus 54 | C28 | + |
| 17 | M. abscessus 67 | C28 | + |
| 18 | M. abscessus 158 | C28 | + |
| 19 | M. abscessus YNKW | C28 | + |
| 24 | M. abscessus LRC18036 | C28 | + |
| 25 | M. abscessus ATCC19977 | T28 | - |
| 26 | M. abscessus JCM15300 | T28 | - |
| 27 | M. abscessus BD | T28 | - |
| 28 | M. chelonae | | - |
| 29 | M. conceptionense | | - |
| 30 | M. fortuitum | | - |
| 31 | M. houstonense | | - |
| 32 | M. salmoniphilium | | - |
| 33 | M. senegalense | | - |
| 34 | M. smegmatis | | - |
| 35 | M. gordonae | | - |
| 36 | M. kansasii | | - |
| 37 | M. tuberculosis T1 | | - |
| 38 | M. tuberculosis T2 | | - |
| 39 | M. tuberculosis T3 | | - |
| 40 | M. tuberculosis T4 | | - |

**[Table 5-2]**

| | | | |
|---|---|---|---|
| 41 | M. tuberculosis T5 | | - |
| 42 | M. tuberculosis T6 | | - |
| 43 | M. tuberculosis T7 | | - |
| 44 | M. tuberculosis T8 | | - |
| 45 | M. tuberculosis T9 | | - |
| 46 | M. tuberculosis T10 | | - |
| 47 | M. avium M1 | | - |
| 48 | M. avium M2 | | - |
| 49 | M. avium M3 | | - |
| 50 | M. avium M4 | | - |
| 51 | M. avium M5 | | - |
| 52 | M. avium M7 | | - |
| 49 | M. avium M8 | | - |
| 50 | M. avium M9 | | - |
| 51 | M. avium M10 | | - |
| 52 | Negative control | | - |

As the result of the assay, it was shown that a strain (C28 strain) of *Mycobacteroides abscessus* complex having erm (41) gene, which has cytosine at position 28, can be specifically detected by the constructed chromatographic detection system, and that there are no cross-reactions with a T28 strain and other mycobacterial species.

### <Example 2>

Primers specifically detecting a C28 strain were designed based on sequence information of the insert nucleotide sequence of SEQ ID NO:2 specifically present in the genomic DNA of a C28 strain. Each primer was designed so that it would specifically hybridize to a partial nucleotide sequence in the insert nucleotide sequence and a nucleic acid amplification reaction takes place when a C28 strain-derived DNA is present in a sample. PCR was performed using the designed primers, and the resultant nucleic acid amplified products were analyzed by agarose gel electrophoresis. In this manner, whether a C28 strain can be specifically detected or not was verified.

**[Table 6]**

| Name | Sequence | SEQ ID NO | Corresponding base in insert nucleotide sequence of SEQ ID NO:2 |
|---|---|---|---|
| 1-C28-1f | GCGCGATGAGACAGGTCACCCG | 18 | 2366-2387 |
| 1-C28-1r | GCAGTCTCTGTGACGGTCAC | 19 | Complementary Sequence of 2682-2701 |
| 1-C28-2f | GACGCAGATACGATGGTGCA | 20 | 2634-2653 |
| 1-C28-2r | TGAAACCGGCTGCTGGTGCGATGTG | 21 | Complementary Sequence of 3549-3573 |
| 2-C28-1f | CTTCAGCGGCAGCTTCAAGC | 22 | 4054-4073 |
| 2-C28-1r | TTGCACCCAATGCGCCACGGGAG | 23 | Complementary Sequence of 5071-5093 |
| 3-C28-1f | CGCACCGTGACGCACAACTCG | 24 | 6545-6565 |
| 3-C28-1r | TCGGCTGCCCGATGGGCCTG | 25 | Complementary Sequence of 6576-6595 |
| 4-C28-1f | TCAAGCTTGGCGATGTACTCGTT | 26 | 7488-7510 |
| 4-C28-1r | CGCCCATTCTCCAAATCGGAAATCAC | 27 | Complementary Sequence of 7941-7966 |
| 4-C28-2f | GCATTGCGTCCTCCTCCGTTCATGG | 28 | 7639-7663 |
| 4-C28-2r | CTCGTTACCCTCGATTTGTTGCCAC | 29 | Complementary Sequence of 8312-8336 |

### (I) PCR

The following PCR reaction solutions were prepared using a second primer set consisting of SEQ ID NO: 18 and 19, a third primer set consisting of SEQ ID NO:20 and 21, a fourth primer set consisting of SEQ ID NO:22 and 23, a fifth primer set consisting of SEQ ID NO:24 and 25, a sixth primer set consisting of SEQ ID NO:26 and 27, and a seventh primer set of SEQ ID NO:28 and 29, separately, in accordance with the manual of TaKaRa Taq HS perfect Mix.

**[Table 7]**

| | |
|---|---|
| 2x TaKaRa TaqHS perfect Mix | 10.0 µl |
| Second to seventh primer set | |
| 5 µM Fw primer | 0.5 µl |
| 5 µM Rv primer | 0.5 µl |
| Template DNA (1 ng/µl) | 1.0 µl |
| Sterile distilled water | 8.0 µl |

Purified genomic DNA fragments prepared separately from *Mycobacteroides abscessus* ATCC19977 and *Mycobacteroides abscessus* LRC18036 were used as DNA templates. *Mycobacteroides abscessus* ATCC19977 is a T28 strain, whereas *Mycobacteroides abscessus* LRC18036 is a C28 strain. If PCR is performed using the second primer set and a genomic DNA of *Mycobacteroides abscessus* LRC18036 as a template, it is estimated that a 336-bp region can be specifically obtained as a nucleic acid amplified product, based on nucleotide sequence information. If PCR is performed using the third primer set in the same manner as above, it is estimated that a 940-bp region can be specifically obtained as a nucleic acid amplified product. If PCR is performed using the fourth primer set in the same manner as above, it is estimated that a 1040-bp region can be specifically obtained as a nucleic acid amplified product. If PCR is performed using the fifth primer set in the same manner as above, it is estimated that a 51-bp region can be specifically obtained as a nucleic acid amplified product. If PCR is performed using the sixth primer set in the same manner as above, it is estimated that a 479-bp region can be specifically obtained as a nucleic acid amplified product. If PCR is performed using the seventh primer set in the same manner as above, it is estimated that a 698-bp region can be specifically obtained as a nucleic acid amplified product. In contrast, if PCR is performed using a genomic DNA of *Mycobacteroides abscessus* ATCC19977, i.e., a T28 strain, as a template, it is estimated that no nucleic acid amplified product is obtained even if any of the second to seventh primer sets is used.

As a negative control, a PCR reaction solution having the same composition as above except that sterilized distilled water (1 µL) was used in place of a template DNA solution as mentioned above, was prepared. A 5 µM Fw primer solution and a 5 µM Rv primer solution were each prepared by dissolving a predetermined primer in sterilized distilled water so as to obtain a concentration of 5 µM.

The PCR reaction solutions were set in a PCR apparatus (LifeEco, by Bioer). After a reaction was carried out at 94°C for one minute, a cycle consisting of a reaction at 94°C for 5 seconds, a reaction at 62°C for 10 seconds and a reaction at 72°C for 5 seconds, was repeated 35 times. After completion of the reaction, the PCR reaction solutions were subjected to agarose gel electrophoresis analysis to detect the presence or absence of a nucleic acid amplified product. The results are shown in Figure 3. Figure 3 shows the detection results of PCR amplified products obtained by using the second primer set (A), the third primer set (B), the fourth primer set (C), the fifth primer set (D), the sixth primer set (E), and the seventh primer set (F), by agarose gel electrophoresis.

As the results of the assay, specific nucleic acid amplified products were observed by agarose gel electrophoresis analysis only when a DNA derived from a C28 strain of *Mycobacteroides abscessus* complex (*Mycobacteroides abscessus* LRC18036) is present in a sample, even if any of the primer sets is used. From the results, it was shown that a C28 strain can be specifically detected by detecting the presence or absence of the insert nucleotide sequence represented by SEQ ID NO:2.

All publications, patents and patent applications cited in the specification are incorporated herein in their entireties by reference.

## Claims

1. A method for determining whether a base corresponding to position 28 in the nucleotide sequence of SEQ ID NO:1 of erm (41) gene is cytosine or thymine in a mycobacterium belonging to *Mycobacteroides abscessus* complex, comprising
detecting, in a genomic DNA of the mycobacterium as a subject to be determined, an indicator nucleotide sequence, which is present in the region except the erm (41) gene in the genomic DNA when the base in the erm (41) gene is cytosine, and which is not present when the base is thymine, wherein
detection of the indicator nucleotide sequence indicates that the base in the erm (41) gene in the genomic DNA of the mycobacterium is cytosine, and
non-detection of the indicator nucleotide sequence indicates that the base in the erm (41) gene in the genomic DNA of the mycobacterium is thymine.

2. The method according to claim 1, wherein the indicator nucleotide sequence is a first nucleotide sequence having consecutive 10 or more nucleotides contained in the nucleotide sequence of SEQ ID NO:2, or a second nucleotide sequence complementary to the first nucleotide sequence.

3. The method according to claim 2, wherein
the first nucleotide sequence at least partially comprises a nucleotide sequence having consecutive 10 or more nucleotides contained in a nucleotide sequence of SEQ ID NO:3, 5, 7, 9 or 11.

4. A primer set for detecting an indicator nucleotide sequence, which is present in the region except erm (41) gene of a genomic DNA when the base corresponding to position 28 in the nucleotide sequence of SEQ ID NO:1 of erm (41) gene is cytosine and which is not present when the base is thymine, in a mycobacterium belonging to *Mycobacteroides abscessus* complex, the primer set comprising:
a first primer comprising a polynucleotide comprising, at the 3' end, a nucleotide sequence f12 capable of hybridizing to a complementary nucleotide sequence to a partial nucleotide sequence f11 having consecutive 10 or more nucleotides which is contained in the indicator nucleotide sequence, and
a second primer comprising a polynucleotide comprising, at the 3' end, a nucleotide sequence r12 capable of hybridizing to a partial nucleotide sequence r11 having consecutive 10 or more nucleotides which is contained in the indicator nucleotide sequence, and positioned closer to the 3' end than the 3' end of the partial nucleotide sequence f11.

5. The primer set according to claim 4, wherein the indicator nucleotide sequence is a third nucleotide sequence having consecutive 20 or more nucleotides contained in the nucleotide sequence of SEQ ID NO:2.

6. The primer set according to claim 5, wherein a nucleotide sequence from the nucleotide at the 5' end of the partial nucleotide sequence f11 to the nucleotide at the 3' end of the partial nucleotide sequence r11 contained in the third nucleotide sequence at least partially comprises a nucleotide sequence having consecutive 20 or more nucleotides contained in a nucleotide sequence of SEQ ID N0:3, 5, 7, 9 or 11.

7. The primer set according to claim 5 or 6, wherein
the partial nucleotide sequence f11 is
(fl 1-12-1) a nucleotide sequence having consecutive 10 or more nucleotides and contained in the nucleotide sequence in the range from position 4624 to position 4668 of the nucleotide sequence of SEQ ID NO:2,
(fl 1-18-1) a nucleotide sequence having consecutive 10 or more nucleotides and contained in the nucleotide sequence in the range from position 2356 to position 2397 of the nucleotide sequence of SEQ ID NO:2,
(f11-20-1) a nucleotide sequence having consecutive 10 or more nucleotides and contained in the nucleotide sequence in the range from position 2624 to position 2663 of the nucleotide sequence of SEQ ID NO:2,
(f11-22-1) a nucleotide sequence having consecutive 10 or more nucleotides and contained in the nucleotide sequence in the range from position 4044 to position 4083 of the nucleotide sequence of SEQ ID NO:2,
(fl 1-24-1) a nucleotide sequence having consecutive 10 or more nucleotides and contained in the nucleotide sequence in the range from position 6535 to position 6575 of the nucleotide sequence of SEQ ID NO:2,
(fl 1-26-1) a nucleotide sequence having consecutive 10 or more nucleotides and contained in the nucleotide sequence in the range from position 7478 to position 7520 of the nucleotide sequence of SEQ ID NO:2, or
(fl 1-28-1) a nucleotide sequence having consecutive 10 or more nucleotides and contained in the nucleotide sequence in the range from position 7629 to position 7673 of the nucleotide sequence of SEQ ID NO:2, and
the partial nucleotide sequence r11 is
(r11-13-1) a nucleotide sequence having consecutive 10 or more nucleotides and contained in the nucleotide sequence in the range from position 4694 to position 4740 of the nucleotide sequence of SEQ ID NO:2,
(r 11-19-1) a nucleotide sequence having consecutive 10 or more nucleotides and contained in the nucleotide sequence in the range from position 2672 to position 2711 of the nucleotide sequence of SEQ ID NO:2,
(r11-21-1) a nucleotide sequence having consecutive 10 or more nucleotides and contained in the nucleotide sequence in the range from position 3539 to position 3583 of the nucleotide sequence of SEQ ID NO:2,
(r11-23-1) a nucleotide sequence having consecutive 10 or more nucleotides and contained in the nucleotide sequence in the range from position 5061 to position 5103 of the nucleotide sequence of SEQ ID NO:2,
(r11-25-1) a nucleotide sequence having consecutive 10 or more nucleotides and contained in the nucleotide sequence in the range from position 6566 to position 6605 of the nucleotide sequence of SEQ ID NO:2,
(r11-27-1) a nucleotide sequence having consecutive 10 or more nucleotides and contained in the nucleotide sequence in the range from position 7931 to position 7976 of the nucleotide sequence of SEQ ID NO:2, or
(r11-29-1) a nucleotide sequence having consecutive 10 or more nucleotides and contained in the nucleotide sequence in the range from position 8302 to position 8346 of the nucleotide sequence of SEQ ID NO:2.

8. The primer set according to any one of claims 4 to 7, wherein one of the first primer and the second primer further comprises a label portion that is a tag capable of binding to a label substance or a label substance; and the other one further comprises a binding portion that is a tag capable of binding to a solid phase support.

9. A kit for detecting an indicator nucleotide sequence, which is present in the region except erm (41) gene of a genomic DNA when the base corresponding to position 28 in the nucleotide sequence of SEQ ID NO:1 of erm (41) gene is cytosine, and which is not present when the base is thymine, in a mycobacterium belonging to *Mycobacteroides abscessus* complex, the kit comprising:
the primer set according to claim 8; and
a solid phase support at least partially comprising a portion capable of binding to the binding portion.

10. A probe for detecting an indicator nucleotide sequence, which is present in the region except erm (41) gene of a genomic DNA when the base corresponding to position 28 in the nucleotide sequence of SEQ ID NO:1 of erm (41) gene is cytosine and which is not present when the base is thymine, in a mycobacterium belonging to *Mycobacteroides abscessus* complex,
the probe comprising a polynucleotide comprising a nucleotide sequence capable of hybridizing to a partial nucleotide sequence p1 having consecutive 10 or more nucleotides and contained in the indicator nucleotide sequence.

11. The probe according to claim 10, wherein the indicator nucleotide sequence is a first nucleotide sequence having consecutive 10 or more nucleotides contained in the nucleotide sequence of SEQ ID NO:2, or a second nucleotide sequence complementary to the first nucleotide sequence.

12. The probe according to claim 11, wherein the partial nucleotide sequence p1 at least partially comprises a nucleotide sequence having consecutive 10 or more nucleotides contained in a nucleotide sequence of SEQ ID NO:3, 5, 7, 9 or 11 or a complementary nucleotide sequence thereof.

13. The probe according to claim 11 or 12, wherein the partial nucleotide sequence p1 is
(p1-12-1) a nucleotide sequence having consecutive 10 or more nucleotides and contained in the nucleotide sequence in the range from position 4624 to position 4668 of the nucleotide sequence of SEQ ID NO:2 or a complementary nucleotide sequence thereof,
(p1-18-1) a nucleotide sequence having consecutive 10 or more nucleotides and contained in the nucleotide sequence in the range from position 2356 to position 2397 of the nucleotide sequence of SEQ ID NO:2 or a complementary nucleotide sequence thereof,
(p1-20-1) a nucleotide sequence having consecutive 10 or more nucleotides and contained in the nucleotide sequence in the range from the position 2624 to position 2663 of the nucleotide sequence of SEQ ID NO:2 or a complementary nucleotide sequence thereof,
(p1-22-1) a nucleotide sequence having consecutive 10 or more nucleotides and contained in the nucleotide sequence in the range from position 4044 to position 4083 of the nucleotide sequence of SEQ ID NO:2 or a complementary nucleotide sequence thereof,
(p1-24-1) a nucleotide sequence having consecutive 10 or more nucleotides and contained in the nucleotide sequence in the range from position 6535 to position 6575 of the nucleotide sequence of SEQ ID NO:2 or a complementary nucleotide sequence thereof,
(p1-26-1) a nucleotide sequence having consecutive 10 or more nucleotides and contained in the nucleotide sequence in the range from position 7478 to position 7520 of the nucleotide sequence of SEQ ID NO:2 or a complementary nucleotide sequence thereof,
(p1-28-1) a nucleotide sequence having consecutive 10 or more nucleotides and contained in the nucleotide sequence in the range from position 7629 to position 7673 of the nucleotide sequence of SEQ ID NO:2 or a complementary nucleotide sequence thereof,
(p1-13-1) a nucleotide sequence having consecutive 10 or more nucleotides and contained in the nucleotide sequence in the range from position 4694 to position 4740 of the nucleotide sequence of SEQ ID NO:2 or a complementary nucleotide sequence thereof,
(p1-19-1) a nucleotide sequence having consecutive 10 or more nucleotides and contained in the nucleotide sequence in the range from position 2672 to position 2711 of the nucleotide sequence of SEQ ID NO:2 or a complementary nucleotide sequence thereof,
(p1-21-1) a nucleotide sequence having consecutive 10 or more nucleotides and contained in the nucleotide sequence in the range from position 3539 to position 3583 of the nucleotide sequence of SEQ ID NO:2 or a complementary nucleotide sequence thereof,
(p1-23-1) a nucleotide sequence having consecutive 10 or more nucleotides and contained in the nucleotide sequence in the range from position 5061 to position 5103 of the nucleotide sequence of SEQ ID NO:2 or a complementary nucleotide sequence thereof,
(p1-25-1) a nucleotide sequence having consecutive 10 or more nucleotides and contained in the nucleotide sequence in the range from position 6566 to position 6605 of the nucleotide sequence of SEQ ID NO:2 or a complementary nucleotide sequence thereof,
(p1-27-1) a nucleotide sequence having consecutive 10 or more nucleotides and contained in the nucleotide sequence in the range from position 7931 to position 7976 of the nucleotide sequence of SEQ ID NO:2 or a complementary nucleotide sequence thereof, or
(p1-29-1) a nucleotide sequence having consecutive 10 or more nucleotides and contained in the nucleotide sequence in the range from position 8302 to position 8346 of the nucleotide sequence of SEQ ID NO:2 or a complementary nucleotide sequence thereof.
